# FASCICULE DE BREVET EUROPEEN

(11) **EP 2 124 982 B1**
(45) Date de publication et mention de la délivrance du brevet: **22.08.2012**
(21) Numéro de dépôt: 07858214.5
(22) Date de dépôt: 28.12.2007
(51) Int. Cl.: A61K 36/36, B01D 11/00, A23L 1/00

(54) **COMPOSITION COMPRENANT UN EXTRAIT DE GRAINES DE QUINOA, UTILISATION DERMATOLOGIQUE**
ZUSAMMENSETZUNG MIT EINEM QUINOA-KORN-EXTRAKT ZUR DERMATOLOGISCHEN ANWENDUNG
COMPOSITION CONTAINING A QUINOA GRAIN EXTRACT FOR DEMATOLOGICAL USE

(30) Priorité: 28.12.2006 FR 0656001
(43) Date de publication de la demande: 02.12.2009
(73) Titulaire: Laboratoires Expanscience, 92400 Courbevoie (FR)
(72) Inventeur: MSIKA, Philippe, F-78000 Versailles (FR)
(74) Mandataire: Faivre Petit, Frédérique
(86) Numéro de dépôt international: PCT/EP2007/064623
(87) Numéro de publication internationale: WO 2008/080974

(56) Documents cités:
- WO-A-99/53933
- WO-A-2005/058249
- WO-A-2006/053415
- ALUKO R E ET AL: "Functional and bioactive properties of Quinoa Seed protein hydrolysates" JOURNAL OF FOOD SCIENCE, INSTITUTE OF FOOD TECHNOLOGISTS, CHICAGO, IL, US, vol. 68, no. 4, 2003, pages 1254-1258, XP003014579 ISSN: 0022-1147
- PRZYBYLSKI R. ET AL.: "Characterization of quinoa (Chenopodium quinoa) lipids." FOOD CHEMISTRY, vol. 51, 1994, pages 187-192, XP002453905
- KOZIOL M.J.: "New crops." 1993, WILEY , NEW YORK , XP002454273 pages 328-336

## Description

L'invention se rapporte à une composition cosmétique, dermatologique ou nutraceutique comprenant un excipient approprié et un extrait de graines de quinoa.

La graine du quinoa est classée parmi les pseudo-céréales en raison de considérations taxinomiques et d'une composition chimique qui la rapproche des graminées. Bien que sa richesse en protéines et en plusieurs minéraux ait été reconnue dès le début du 20^{ème} siècle, sa consommation est restée cantonnée à la Cordillère des Andes pendant près de 6 000 ans. Dans la langue Quechua d'origine Incas, le mot quinoa signifie "chisiya mama" ou "graine mère" en espagnol. Aux 18^{ème} et 19^{ème} siècles, les botanistes voyageurs qui traversèrent les régions andines en faisaient grande éloge. Les tentatives de cultures qu'ils firent en Europe furent peu concluantes. Un agronome Allemand prétendit en 1917 avoir réussi des essais mais ceux-ci tombèrent dans l'oubli. En raison d'un intérêt nutritionnel avéré, de nombreuses recherches menées ces trente dernières années et de la curiosité qu'elle suscite, la graine du quinoa connaît un attrait grandissant. Le nom botanique du quinoa est *Chenopodium quinoa* Willd. subsp. *quinoa.* Ses noms usuels et vernaculaires sont :
- en quechua: quinua, kiuna ; (il existe de nombreux autres noms locaux en Amérique du Sud) ;
- en espagnol : quinoa, quinua, arroz del Peru ;
- en français : quinoa, petit riz, riz du Pérou ;
- en anglais : quinoa, quinua.

*Chenopodium quinoa* est une plante herbacée annuelle mesurant 0,5 à 2,1 mètres de haut selon les conditions environnementales et le génotype (plants cultivés 1 à 1,5 mètre). La racine pivot est densément ramifiée ce qui facilite sa résistance au gel. La partie aérienne apparaît ramifiée ou pas selon les variétés. Ses feuilles, alternes, sont assez polymorphes (lancéolées, deltoïdes ou triangulaires). Elles sont vertes lorsque la plante est encore jeune puis prennent une teinte jaune, rouge ou pourpre à maturité. L'inflorescence est de type panicule. Les fleurs apétales sont petites et sessiles.

Le fruit indéhiscent est un akène. Il renferme de petites graines presque sphériques mesurant de 1 à 2,0-2,6 mm de diamètre et dont l'allure rappelle celle du millet. Leur couleur est blanche, jaune, rouge, pourpre, brune ou noire. Le péricarpe représente environ 8% de la graine, l'embryon 60-69%, et le périsperme environ 23%.

Parmi le genre *Chenopodium,* deux espèces sauvages, *C. hircinum* et *C. berlandieri,* sont proches de *Chenopodium quinoa* (même nombre de chromosomes (2n = 36) et hybridations interspécifiques). Des cas d'hybridations ont aussi été observés entre quinoa et chénopode blanc (*Chenopodium album L*.).

Les quinoas cultivés présentent d'importantes variabilités de couleurs (plantes, inflorescences, graines), de teneurs en protéines et en saponines dans les graines, et de teneurs en beta-cyanine et en oxalates de calcium dans les feuilles.

La région andine, et plus particulièrement les bords du lac Titicaca, s'avèrent d'une grande diversité génétique de population. Les principales variétés connues dans cette région sont les suivantes :
- au Pérou : Kancolla, Cheweca, Witulla, Tahuaco, Camacani, Yocarà, Wilacayuni, Blanca de Juli, Amarilla de Marangani, Pacus, Rosada, Blanca de Junin, Hualhuas, Huancayo, Mantaro, Huacariz, Huacataz, Acostambo, Blanca Ayacuchana et Nariño ;
- en Bolivie : Sajama, Real Blanca, Chucapaca, Kamiri, Huaranga, Pasancalla, Pandela, Tupiza, Jachapucu, Wila Coymini, Kellu, Uthusaya, Chullpi, Kaslali et Chillpi ;
- en Equateur : Inbaya, Chaucha, INIAP-Cochasqui, Tanlahua, Piartal, Porotoc, Amarga del Chimborazo, Amarga de Imbabura et Morada ;
- en Colombie, Dulce de Quitopampa ;
- en Argentine : Blanca de Jujuy ;
- au Chili : Baer, Lito, Faro et Picchaman.

Un botaniste bolivien a décrit en 1968 17 races d'après leurs caractères morphologiques. D'autres botanistes ont proposé quatre principaux éco-types définis d'après leur localisation géographique : le type « Vallée », typique de 2 000 à 4 000 m d'altitude; le type "Altiplano", typique des hautes terres situées au dessus de 4 000 m ; le type "Salé" situé vers 4 000 m mais adapté aux forts pH des sols de la région d'Atacama ; et les types "Niveau de la mer" rencontrés dans les vallées intérieures de la Bolivie. Le quinoa sauvage a pour origine les hauts plateaux de la Cordillère des Andes. Il se rencontre au Pérou, en Bolivie et à l'extrême Nord du Chili à des altitudes pouvant dépasser 3 900 mètres.

Les graines sèches de quinoa sont composées d'eau (environ 10%, valeur de 12,6% rapportée dans la littérature), de matières minérales (valeurs de 2,46 à 3,4 % rapportées dans la littérature), de glucides (valeurs de 58,5% et de 61,2% rapportées dans la littérature pour les graines brutes, et de 62,8% pour les graines polies), de protéines (valeurs de 12,2 à 13,8% rapportées dans la littérature, 14,8 et 15,7% pour quinoas sucrés et amères respectivement), des lipides (valeurs variant d'environ 4,5 à environ 10% rapportées dans la littérature), des saponosides et des polyphénols. Les pourcentages indiqués sont exprimés en poids par rapport au poids total de la graine sèche.

En tant que glucides, la graine sèche de quinoa comprend des fibres alimentaires (valeur 6,6% rapportée dans la littérature), des fibres brutes (valeur de 2,2% rapportée dans la littérature) dont des fibres solubles dans l'eau (1,26 g/100 g d'après la littérature) et des fibres insolubles dans l'eau (5,38 g/100 g d'après la littérature). Le glucose (4,5% d'après la littérature), le fructose (2,4% d'après la littérature) et le saccharose (2,4% d'après la littérature) sont importants. Les pourcentages indiqués sont exprimés en poids par rapport à la teneur totale en poids de glucides dans la graine sèche.

En tant que lipides, la graine sèche de quinoa comprend des acides gras, des phosphatides (lysophosphatidyléthanolamine principalement), des tocophérols (α-tocophérol et γ-tocophérol principalement), des hydrocarbures (squalène) et des stérols.

Les acides gras majoritaires sont l'acide linoléique et l'acide oléique. La graine sèche contient également des quantités significatives d'acide palmitique et d'acide α-linolénique. Elle peut également contenir de l'acide myristique, de l'acide 5-hexadécénoïque, de l'acide stéarique, de l'acide arachidique, de l'acide eicasénoïque, de l'acide béhénique, de l'acide 9-docosénoïque, de l'acide tétracosénoïque ou d'autres acides. Les graines brutes et les graines polies et lavées ont des proportions en acides gras très proches.

Dans la littérature, on a rapporte des taux d'acides gras libres élevés : 18,9% dans la graine entière ; un indice d'iode de 129 ; un indice de saponification de 190, une teneur en matière insaponifiable de 5,2%.

D'après la littérature_{;} les stérols majoritaires sont le Δ7-stigmastérol. Les autres stérols pouvant être présent sont le Δ5,24 (28)-avenastérol, le β-sitostérol, le Δ7-campestérol, le stigmastérol, le cholestérol, le campestérol, le fucostanol, le 24-éthylène-Δ7-cholesten-3β-ol.

Les saponosides présents dans la graine de Quinoa ont été beaucoup étudiés depuis 25 ans par des chercheurs de différents continents. Il s'agit de saponosides triterpéniques. Ces constituants sont essentiellement situés au niveau du péricarpe de la graine.

Les applications du quinoa, et de ses extraits à l'exception des saponines, ce sont, jusqu'à présent, cantonnées au domaine alimentaire. A titre d'exemple, on peut citer la demande internationale WO2005/058249 qui décrit un concentrat de protéines utilisé dans l'alimentaire.

La demande internationale WO2006/05341 divulgue des utilisations dermatologiques de nombreux extraits de plantes dont le quinoa. Les extraits de quinoa sont obtenus par extraction aqueuse ou éthanolique sur plantes stressées et non stressées. L'extrait aqueux ainsi obtenu contient quelques protéines hydrosolubles, des tanins, des sucres libres et des oligosaccharides, des hétérosides (saponines + flavoniques). L'extrait éthanolique contient, quant à lui, des saponines, des polyphénols, des lipides triterpéniques et quelques lipides. Toutefois, les tests réalisés sur l'interleukine IL-8 montrent que l'extrait testé induit la synthèse de cette interleukine IL-8 et il faut donc en conclure que cet extrait est pro-inflammatoire. Ainsi, il n'est, en fait, pas recommandé pour une utilisation dermatologique. Aucune utilisation dermatologique sérieuse du quinoa et de ses extraits n'a donc à ce jour été envisagée.

Or, les inventeurs ont découvert, de manière surprenante, que certains extraits de quinoa ne sont pas inflammatoires et présentent des propriétés cosmétiques, dermatologiques ou nutraceutiques intéressantes.

Il est ici divulgué une composition cosmétique, dermatologique ou nutraceutique comprenant un extrait de graines de quinoa et le cas échéant un excipient approprié, caractérisée en ce que ledit extrait est un extrait peptidique ou un extrait lipidique de graines de quinoa. La composition nutraceutique peut ne pas comprendre d'excipient.

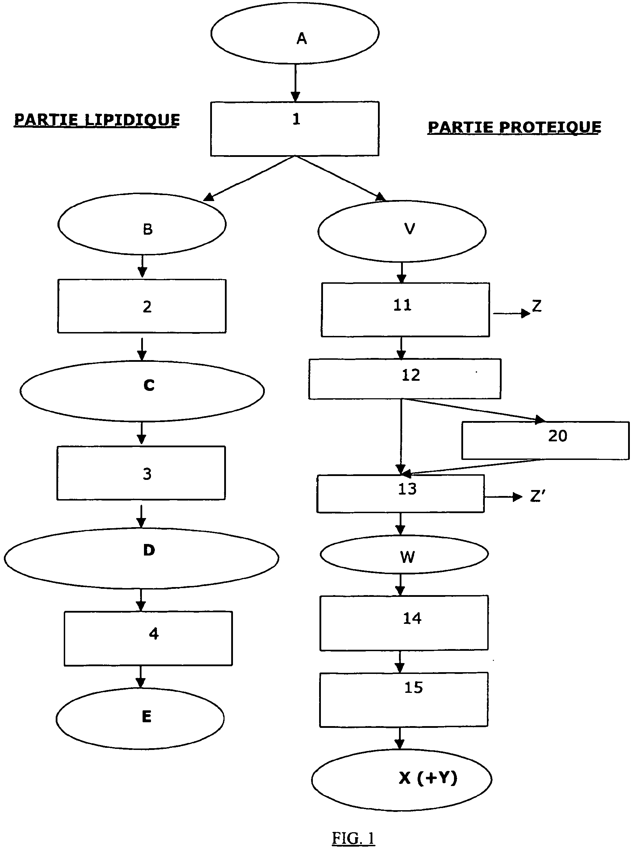

La figure 1 identifie les différentes étapes d'obtention des divers extraits lipidiques et peptidiques. A partir de graines de quinoa (A), le procédé comprend une première étape (1) d'extraction par pression, par solvant, sous pression supercritique. De cet extrait, est valorisée soit la partie lipidique (huile brute B) soit la partie peptidique (tourteau V). L'huile brute est soumise à un raffinage (2) pour conduire à une huile raffinée (C). Cette huile raffinée (C) est soumise à une distillation moléculaire (3) pour conduire à une huile concentrée en sa fraction insaponifiable (ou concentrât ; D). Cette huile concentrée (D) est ensuite soumise à une saponification et extraction (4) pour conduire aux insaponifiables (E). En ce qui concerne la partie protéique, le tourteau (V) est lavé à l'eau et/ou éthanol (11) afin de supprimer les saponines, sucres solubles, hétérosides et polyphénols (Z). Le tourteau lavé est ensuite soumis à une étape de solubilisation des protéines à pH alcalin (12). A titre d'alternative, le procédé peut comprendre une étape supplémentaire de traitement enzymatique α -amylases / cellulases (20). Le procédé comprend ensuite une étape de centrifugation puis ultrafiltration (13), ce qui permet l'élimination des insolubles (Z') et conduit aux protéines concentrées (W). ces protéines concentrées (W) sont ensuite soumises à une étape de traitement enzymatique par protéases (14) puis traitement thermique, ultrafiltration et nanofiltration (15), ce qui conduit aux peptides (X) (+ sucres Y).

La présente invention a tout d'abord pour objet une composition cosmétique, dermatologique ou nutraceutique comprenant un extrait lipidique de graines de quinoa et le cas échéant un excipient approprié, ledit extrait lipidique de quinoa étant un insaponifiable.

Les huiles de graines de quinoa peuvent être extraites par plusieurs procédés :
- extraction physique telle que la pression à froid sur presse mécanique, la pression sur extrudeuse bi-vis ;
- extraction chimique à l'aide de solvants organiques (alcanes aliphatiques, alcools, solvants chlorés, solvants fluorés) ;
- extraction en milieu supercritique, à l'aide du dioxyde de carbone seul et/ou avec des co-solvants.

Avant extraction de l'huile, les saponines, contenues majoritairement sur la paroi externe des graines, sont avantageusement préalablement éliminées par décorticage abrasif ou par lavage à l'eau. Par ailleurs, les graines peuvent préalablement être traitées hydro-thermiquement.

Pour l'extraction d'huile brute de Quinoa, l'utilisation d'un solvant chimique de type alcane aliphatique tel que le n-hexane sera préférée. Selon un mode d'extraction particulier, les graines, décortiquées et lavées, éventuellement pré-traitées hydro-thermiquement, sont aplaties, afin d'obtenir des flocons, avant d'être introduites dans un extracteur continu à bande, et les lipides sont extraits par percolation de n-hexane. Le miscella recueilli est évaporé sous vide afin de récupérer l'huile désolvantée.

Les spécifications de l'huile brute de Quinoa sont données dans le tableau 1 suivant :

**Tableau 1: spécifications d'huile de Quinoa brute**

| Coupe grasse (% en poids par rapport au poids total de l'huile) | |
|---|---|
| C14 (acide myristique) | ≤ 1,0 |
| C16 (acide palmitique) | 5,0 -12,0 |
| C16' (acide 5-hexadécénoïque) | ≤ 1,0 |
| C 18 (acide stéarique) | ≤ 2,0 |
| C18' (acide oléique) | 20,0 -35,0 |
| C18" (acide linoléique) | 40,0 - 60,0 |
| C18"' (acide α-linolénique) | 2,0 - 13,0 |
| C20 (acide arachidique) | ≤ 1,0 |
| C20' (acide eicasénoïque) | ≤ 3,0 |
| C22 (acide béhénique) | ≤ 1,0 |
| C22' (acide 9-docosénoïque) | ≤ 3,0 |
| C24 (acide tétracosénoïque) | ≤ 1,0 |
| Teneur en Tocophérols (mg/100g) | 4-300 |
| Teneur en Stérols (g/100g) | 1,0 - 3,0 |
| Teneur en Squalène (g/100 g) | 1,0 - 5,0 |
| Teneur en Insaponifiable (g/100 g) | 3,0 - 8,0 |

Cette huile est alimentaire. A ce titre, elle peut être consommée par l'homme. Elle respecte donc les normes CODEX. Elle ne contient pas ou très peu d'acides gras libres. D'après les normes en vigueur, les valeurs maximales d'indice d'acide sont de 0,4 mg KOH/g d'huile, pour les huiles obtenues par pression à froid et les huiles vierges. Elle ne contient pas de sous produits d'oxydation ou de produits résiduels du type résidus phytosanitaires et HAPs (hydrocarbures aromatiques polycyclique).

L'huile brute de Quinoa peut être raffinée selon des procédés connus de l'homme de métier tels que le raffinage physique (dégommage à l'eau, désacidification par désodorisation à haute température) et le raffinage chimique (démucilagination à l'eau ou traitement acide afin d'éliminer les phospholipides, neutralisation des acides gras libres à l'aide d'une solution basique, décoloration, frigelisation et désodorisation). Le raffinage chimique sera préféré car il permet d'éliminer les saponines entraînées lors de l'extraction, ainsi que la forte proportion de phospholipides et d'acides gras libres.

Les spécifications de l'huile de Quinoa raffinée sont données dans le tableau 2 suivant :

**Tableau 2: spécifications d'huile de Quinoa raffinée**

| Coupe grasse (% en poids par rapport au poids total de l'huile) | |
|---|---|
| C14 | ≤ 1,0 |
| C16 | 5,0 -12,0 |
| C16' | ≤ 1,0 |
| C18 | ≤ 2,0 |
| C18' | 20,0-35,0 |
| C18" | 40,0 60,0 |
| C18"' | 2,0 - 13,0 |
| C20 | ≤ 1,0 |
| C20' | ≤ 3,0 |
| C22 | ≤ 1,0 |
| C22' | ≤ 3,0 |
| C24 | ≤ 1,0 |
| Teneur en Tocophérols (mg/100g) | 4 - 200 |
| Teneur en Stérols (g/100g) | 0,5 - 3,0 |
| Teneur en Squalène (g/100 g) | 0,5 - 3,0 |
| Teneur en Insaponifiable (g/100 g) | 2,0 - 6,0 |

Cette huile est alimentaire. A ce titre, elle peut être consommée par l'homme. Elle respecte donc les normes CODEX. Elle ne contient pas ou très peu d'acides gras libres. D'après les normes en vigueur, les valeurs maximales d'indice d'acide sont de 0,6 mg KOH/g d'huile, pour les huiles raffinées. Elle ne contient pas de sous produits d'oxydation ou de produits résiduels du type résidus phytosanitaires et HAPs (hydrocarbures aromatiques polycyclique). Elle présente également des triglycérides dont la répartition en acides gras est identique à l'huile de départ, ce qui lui permet de bénéficier de l'appellation huile végétale naturelle.

Il est également divulgué un procédé de préparation d'une huile raffinée de quinoa ayant des spécifications définies dans le tableau 2, comprenant une étape de raffinage chimique de l'huile brute de quinoa. Selon une variante avantageuse, le procédé comprend les étapes décrites précédemment.

L'huile raffinée de quinoa obtenue précédemment peut être concentrée en sa fraction insaponifiable par un procédé de distillation moléculaire.

L'insaponifiable est la fraction d'un corps gras qui, après action prolongée d'une base alcaline, reste insoluble dans l'eau et peut être extraite par un solvant organique. Cinq grands groupes de substances sont présents dans la plupart des insaponifiables d'huiles végétales : hydrocarbures saturés ou insatures, alcools aliphatiques ou terpéniques, stérols, tocophérols, les pigments caroténoides et xanthophylles.

Cette étape de distillation moléculaire est de préférence réalisée en utilisant un dispositif choisi parmi les distillateurs moléculaires de type centrifuge et les dispositifs moléculaires de type à film raclé.

Les distillateurs moléculaires de type centrifuge sont connus de l'homme de métier. Par exemple, la demande EP 493 144 décrit un distillateur moléculaire de ce type. D'une manière générale, le produit à distiller est étalé en couche mince sur la surface chauffée (surface chaude) d'un rotor conique tournant à grande vitesse. L'enceinte de distillation est placée sous vide. Dans ces conditions, il y a évaporation et non pas ébullition, depuis la surface chaude, des constituants de l'huile tels que les insaponifiables, l'avantage étant que l'huile et ses constituants, notamment les insaponifiables (ces produits étant réputés fragiles), ne sont pas dégradés au cours de l'évaporation.

Les distillateurs moléculaires de type à film raclé sont également connus de l'homme du métier. D'une manière générale, ils comprennent une chambre de distillation dotée d'un racleur tournant, permettant l'étalement en continu sur la surface d'évaporation (surface chaude) des produits à distiller. Les vapeurs de produit sont condensées par le biais d'un doigt réfrigéré, placé au centre de la chambre de distillation. Les systèmes périphériques d'alimentation et de vide sont très proches de ceux d'un distillateur centrifuge (pompes d'alimentation, pompes à vide à palette et à diffusion d'huile, etc.). La récupération des résidus et des distillats dans des ballons en verre se fait par écoulement gravitationnel.

A l'issue de l'étape de fractionnement, la fraction distillée riche en insaponifiables représente avantageusement 5 à 15 % en poids de l'huile de départ, et la fraction distillée riche en triglycérides représente avantageusement 85 à 95 % en poids de l'huile de départ. Il a en outre été vérifié que ce procédé n'entraînait aucune modification chimique ou altération des composés de l'insaponifiable, et que les fractions fortement insaturées étaient préservées. Par conséquent, la répartition en acides gras de l'huile de quinoa concentrée est identique à celle de l'huile de quinoa avant concentration.

, l'huile de quinoa concentrée en sa fraction insaponifiable présente les spécifications données dans le tableau 3 suivant :

**Tableau 3: spécifications d'une huile raffinée concentrée en sa fraction insaponifiable**

| Coupe grasse (% en poids par rapport au poids total de l'huile) | |
|---|---|
| C14 | ≤ 1,0 |
| C16 | 5,0 -15,0 |
| C16' | ≤ 1,0 |
| C18 | ≤ 2,0 |
| C18' | 20,0 -35,0 |
| C18" | 40,0 - 60,0 |
| C18'" | 2,0 - 13,0 |
| C20 | ≤ 1,0 |
| C20' | ≤ 3,0 |
| C22 | ≤ 1,0 |
| C22' | ≤ 3,0 |
| C24 | ≤ 1,0 |
| Teneur en Tocophérols (mg/100g) | 40 - 5000 |
| Teneur en Stérols (g/100g) | 3,0 - 20,0 |
| Teneur en Squalène (g/100 g) | 2,0 - 40,0 |
| Teneur en Insaponifiable (g/100 g) | 5,0 - 50,0 |

Cette huile raffinée enrichie en sa fraction insaponifiable est en elle-même un nouvel aliment, divulgué ici. A ce titre, elle peut être consommée par l'homme. Elle respecte donc les normes CODEX. Elle ne contient pas ou très peu d'acides gras libres. Dans le cadre d'une procédure d'agrément pour un nouveau produit alimentaire (« novel food ») devant les instances compétente, des valeurs maximales d'indice d'acide seront définies.

En outre, elle ne contient pas de sous produits d'oxydation ou de produits résiduels du type résidus phytosanitaires et HAPs (hydrocarbures aromatiques polycyclique). Elle présente également des triglycérides dont la répartition en acides gras est identique à l'huile de départ, ce qui lui permet de bénéficier de l'appellation huile végétale naturelle.

Cette huile raffinée étant enrichie en sa fraction insaponifiable, elle permet d'apporter à l'organisme, pour un même apport en triglycérides qu'une huile raffinée, des quantités plus importantes d'éléments nutritifs, tels que des phytostérols et des vitamines, sans apport calorifique supplémentaire.

Il est également divulgué un procédé de préparation d'une huile de quinoa concentrée en sa fraction insaponifiable, comprenant une étape de distillation moléculaire d'une huile raffinée de quinoa. En particulier, l'étape de distillation moléculaire est réalisée en utilisant un dispositif choisi parmi les distillateurs moléculaires de type centrifuge et les dispositifs moléculaires de type à film raclé. Le procédé comprend avantageusement les étapes décrites précédemment.

L'insaponifiable d'huile de quinoa peut être obtenu par des procédés connus de l'homme de métier. Par exemple, il peut être obtenu en effectuant une saponification sur l'huile de quinoa concentrée en sa fraction insaponifiable, puis en extrayant cet insaponifiable à l'aide d'un solvant approprié. Cet extrait est ensuite lavé jusqu'à élimination complète des savons puis le solvant est évaporé. Enfin l'insaponifiable subit avantageusement une désodorisation à la vapeur d'eau puis stripping à l'azote afin d'éliminer les traces de solvant

L'insaponifiable d'huile de Quinoa présente avantageusement les spécifications données dans le tableau 4 suivant :

**Tableau 4: spécifications d'un insaponifiable d'huile de Quinoa**

| | |
|---|---|
| Teneur en Tocophérols (g/100g) | 1,5-3,5 |
| Teneur en Stérols (g/100g) | 20,0 - 50,0 |
| Teneur en Squalène (g/100 g) | 20,0 - 50,0 |

L'invention se rapporte aussi à un procédé de préparation d'un insaponifiable de quinoa, comprenant une étape de saponification d'une huile de quinoa concentrée en sa fraction insaponifiable, puis une extraction de cet insaponifiable à l'aide d'un solvant approprié. Le procédé comprend avantageusement les étapes décrites précédemment

La présente invention a également pour objet une composition telle que définie précédemment comprenant un extrait lipidique de graines de quinoa qui est lui-même choisi dans le groupe constitué par une huile concentrée en sa fraction insaponifiable, un insaponifiable ou une huile raffinée ayant les spécifications données précédemment (tableau 2) pour son utilisation dans la prévention et le traitement des réactions ou pathologies allergiques, inflammatoires, irritativesde la peau et/ou des muqueuses et/ou des phanères immatures, normales ou matures.

Il est également divulgué ici un extrait peptidique et osidique de graines de quinoa.

L'extrait peptidique et osidique est avantageusement obtenu par un procédé comprenant les étapes successives suivantes :
a) à partir de graines de quinoa, extraction d'une huile brute et d'un tourteau et récupération dudit tourteau ;
b) lavage dudit tourteau par l'eau ou un mélange hydroalcoolique pour ne conserver que la partie protéique, puis
c) solubilisation des protéines ;
d) concentration des protéines puis hydrolyse desdites protéines en peptides ;
e) purification et récupération de l'extrait peptidique.

L'extrait peptidique et osidique selon l'invention présente avantageusement les spécifications suivantes :

**Tableau 5: spécifications d'un extrait peptidique de Quinoa**

| % en poids par rapport au poids total de l'extrait peptidique | |
|---|---|
| Teneur en peptides (%) | 25 - 90 |
| Teneur en sucres totaux (%) | 10 - 50 |

Il est également divulgué ici un procédé de préparation d'un extrait peptidique et osidique de quinoa, comprenant les étapes successives suivantes :
a) à partir de graines de quinoa, extraction d'une huile brute et d'un tourteau et récupération dudit tourteau ;
b) lavage dudit tourteau par l'eau ou un mélange hydroalcoolique pour ne conserver que la partie protéique, puis
c) solubilisation des protéines ;
d) concentration des protéines puis hydrolyse desdites protéines en peptides ;
e) purification et récupération de l'extrait peptidique.

Par ailleurs, préalablement à la concentration des protéines (étape d)), les fibres sont avantageusement éliminées

Un mode de réalisation préféré d'obtention de l'extrait peptidique et osidique est décrit ci-après.

Le tourteau de graines de Quinoa obtenu après désolvantation, lors de l'extraction des lipides, est dispersé dans l'eau ou dans un mélange éthanol / eau afin d'extraire et d'éliminer les saponosides, les hétérosides et les polyphénols. Ce mélange est essoré ou centrifugé afin de récupérer le culot et le jus est écarté. Le culot est dispersé et mélangé dans l'eau, à un pH alcalin compris entre 8 et 13, afin de solubiliser les protéines. Il est possible soit d'éliminer les fibres par une nouvelle centrifugation, soit de pratiquer une hydrolyse de l'amidon et des fibres (cellulose, hémicellulose, ...) à l'aide d'un mélange d'α-amylases et de cellulases.

Les protéines solubles sont alors concentrées soit par précipitation en milieu acide au point isoélectrique, soit par ultrafiltration. Les protéines concentrées sont ensuite hydrolysées par des enzymes, avantageusement des protéases alcalines. Un traitement thermique permet de dénaturer les enzymes en fin de réaction.

Le milieu réactionnel subit une ultrafiltration sur une membrane ayant un seuil de coupure de 10 kDa afin d'éliminer les protéines résiduelles (retentât). Le perméat est ensuite concentré au taux de matière sèche désiré et dessalé par nanofiltration avec une membrane de seuil de coupure 200 Da. Enfin le produit est conditionné après avoir été filtré stérilement (0,2 µm).

La composition peut en outre comprendre au moins un composé choisi dans le groupe constitué par
- des actifs classiquement utilisés en dermatologie tels que les émollients, les actifs hydratants, les activateurs de la synthèse de kératine, les kératorégulateurs, les kératolytiques, les agents restructurant de la barrière cutanée (activateurs de la synthèse des lipides cutanés, des agonistes PPARs ou Peroxysome Proliferator Activated Receptor),les agonistes RXR ou LXR , les SERM, les agonistes des récepteurs à la vitamine D ou aux corticoïdes, les activateurs de la différenciation des kératinocytes (rétinoïdes, calcidone®, le calcium), les sébo-régulateurs (les inhibiteurs de 5-alpha réductase, notamment l'actif 5-alpha Avocuta® commercialisé par les Laboratoires Expanscience), les conservateurs, les agents anti-irritants, les agents apaisants, les filtres et écrans solaires, les agents anti-oxydants,
- des principes actifs ayant une action thérapeutique complémentaire, tels que les antibiotiques, les pré et probiotiques, les agents anti-bactériens, les composés antifongiques, les agents anti-viraux, les immunomodulateurs (tacrolimus ou pimécrolimus), les oxazolines, les facteurs de croissance, les agents cicatrisants ou les molécules eutrophiques, les médicaments, les agents anti-inflammatoires, les agents pigmentants ou hypopigmentants, les agents lipolytiques ou inhibiteurs de la lipogénèse, les filtres et écrans solaires minéraux ou organiques pigmentaires ou ultrafins, des aliments classiques ou fonctionnels : hyper ou hypoglycémiants, des nutriments anti-graisse ou anticellullite, anti-cholestérol, anti-oxydant, énergisant, reconstituant, ayant un impact sur les signes secondaires de la ménopause,
- des extraits naturels de plante (parties de végétaux extractibles en phase aqueuse ou huileuse : polyphénols, flavonoides , autres peptides et sucres, .....), des composés contenant des insaponifiables d'huiles végétales, des insaponifiables stéroliques ou des produits pouvant en contenir (insaponifiables d'huiles végétales, notamment insaponifiables d'huile de soja, insaponifiables de beurres végétaux ou de matières butyreuses et leurs mélanges, insaponifiables de cires naturelles, insaponifiables d'extraits huileux, insaponifiables de co-produits huileux industriels, insaponifiables d'extraits de corps gras d'origine animale, insaponifiables d'huiles marines, insaponifiables d'extraits de la matière grasse lactique, insaponifiables de lipides extraits d'organismes unicellulaires, insaponifiables des lipides extraits des algues et organismes marins, etc), des stérols, des stanols, des phytostérols, des phytostanols, des tocophérols, des concentrats d'huiles de tournesol, de colza et/ou de palme, des oligo-éléments, des vitamines, des acides gras en oméga 3, 6 ou 9, des plantes hypoglycémiantes ou hyperglycémiantes ou sucrantes.

Les activateurs de la synthèse de kératine pouvant être utilisés en association sont avantageusement les rétinoïdes, les peptides de lupin commercialisés par la société Silab, des protéines clés du stratum corneum ou granulosum (kératines) et des cornéodesmosomes.

Les agents apaisants pouvant être utilisés en association sont avantageusement l'alpha bisabolol, les dérivés de réglisse, l'ibuprofène, l'enoxolone. Les kératorégulateurs pouvant être utilisés en association sont avantageusement les alpha hydroxy acides et leurs dérivés. Un kératolytique pouvant être utilisé en association est notamment l'acide salicylique et ses dérivés.

Les facteurs de croissance pouvant être utilisés en association sont avantageusement la becaplermine et le TGF-beta (Transforming Growth Factor beta), l'EGF, le NGF, le VEGF.

Les antioxydants pouvant être utilisés en association sont avantageusement choisis dans le groupe constitué par les oligo-éléments (cuivre, zinc, sélénium), l'acide lipoïque, seul ou associé à la vitamine B12, les vitamines C, les vitamines E, les flavonoïdes (thé vert,..), le beta-carotène, le lycopène ou la lutéine, les substances anti-glycation telles que la carnosine, la n-acetyl-cystéine, les isoflavones de soja, les protéines de soja, ainsi que les enzymes anti-oxydants ou radicalaires SOD (super oxyde dismutase) catalase, gluthathion peroxydase, thioredoxine reductase et leurs agonistes.

Les agents restructurant de la barrière cutanée, permettant de stimuler la synthèse des lipides clés de l'épiderme, et pouvant être utilisés en association sont avantageusement des concentrats de tournesol, plus avantageusement des concentrats de tournesol linoléiques, tels que l'actif commercialisé par les Laboratoires Expanscience, Soline® (cf. la demande internationale WO 01/21150), des insaponifiables d'huile végétale, tel que l'Avocadofurane® (cf. la demande internationale WO 01/21150), des agonistes PPARs (rosiglitazone, pioglitazone), RXR, LXR.

Les composés antifongiques pouvant être utilisés en association sont avantageusement l'econazole et le ketoconazole.

Les conservateurs antiseptiques pouvant être utilisés en association sont par exemple le triclosan, la chlorhéxidine, les ammoniums quaternaires.

Les antibiotiques pouvant être utilisés en association sont avantageusement l'acide fucidique, la pénicilline, les tétracyclines, la pristinamycine, l'érythromycine, la clindamycine, la mupirocine, la minocycline, la doxycycline.Les agents anti-viraux pouvant être utilisés en association sont avantageusement l'acyclovir et le valacyclovir. Les agents anti-irritants pouvant être utilisés en association sont avantageusement la glycine, les sucres et/ou peptides de lupin, le Cyclocérarnide®(dérivé d'oxazoline).

Les agents cicatrisants pouvant être utilisés en association sont avantageusement la vitamine A, le panthénol, l'Avocadofurane®, l'oxyde zinc, le magnésium, le silicium, l'acide madécassique ou asiatique, le sulfate de dextran, la glucosamine, la chondroïtine sulfate et globalement les GAG, les peptides de soja fermenté ou non , les oligo-éléments.

Les médicaments pouvant être utilisés en association sont avantageusement les médicaments, appropriés pour une administration pour voie topique ou orale, pour la prévention et/ou le traitement de l'atopie (corticoïdes, immunomodulateurs topiques inhibiteurs de la calcineurine, émollients), de l'acné (antibiotiques, péroxyde de benzoyle, rétinoïdes, acide azélaïque, vitamine PP,vitamine B3, zinc, cyclines), de l'eczéma (immunomodulateurs, émollients, huile de saumon, de bourrache, les prébiotiques) ou du psoriasis (corticoïdes, calcipotriol, calcitriol, tazarotène, huile de cade, acitrétine, PUVA thérapie) ou des médicaments (ou aliments) hyper lipémiants et/ou des médicaments (ou aliments) hypolipémiants. Parmi ces deux derniers types de médicaments, il est possible de citer, les médicaments à base de sulfonylurées et de glinides, les médicaments à base d'inhibiteurs des alpha-glucosidases, les médicaments à base de biguanides (metformine), les médicaments à base d'activateurs de la sensibilité à l'insuline ou thiazolidinediones (TZD, pioglitazone, rosiglitazone), qui sont des agonistes PPARs, les médicaments hypolipémiants de la famille des statines ou de la famille des fibrates (agonistes de PPARα), l'orlistat (Xenical) et la sibutramine (Réductyl ou Sibutral).

Les nutriments anti-graisse pouvant être utilisés en association sont avantageusement choisis dans le groupe constitué par des nutriments bloqueur de l'absorption des graisses, tels que le chitosan, des nutriments capables d'augmenter la thermogénèse (« brûleur de graisse ») tels que l'éphédrine (herbe chinoise Ma Huang), la caféine, la théine et le citrus aurantium, des nutriments capables de réguler l'appétit (« coupe faim ») tels que la L-phénylalanine et la L-tyrosine, des nutriments capables de réguler la glycémie, tels que des minéraux, par exemple le chrome ou le vanadium ou le magnésium, ou l'herbe ayurvédique Gymnema sylvestre, des inhibiteurs de la lipogénèse, tele que l'acide hydroxycitrique extrait du Garcinia cambodgia et des nutriments capables de transporter des graisses tels que la L-carnitine.

Des exemples d'aliments ou de thérapies hyper glycémiantes, pour rééquilibrer la glycémie, sont les antirétrovirus, les glucocorticoïdes, les immunosuppresseurs, IFN-Alpha, les stéroïdes sexuels, le THS, la pilule, les hormones de croissance, les sympathomimétiques, les médicaments cardio-vasculaires, les diurétiques, les Bêtabloquants, les inhibiteurs calciques, les psychotropes.

Les agents anti-inflammatoires pouvant être utilisés en association sont avantageusement des agents anti-inflammatoires stéroïdiens (AIS), tels que les corticoïdes, ou non-stéroïdiens (AINS).

Les immnumodulateurs pouvant être utilisés en association sont avantageusement le tacrolimus, le pimécrolimus et les oxazolines. Les oxazolines pouvant être utilisées en association sont avantageusement des oxazolines choisies dans le groupe constitué par la 2-undécyl-4-hydroxyméthyl-4-méthyl-1,3-oxazoline, la 2-undécyl-4,4-diméthyl-1,3-oxazoline, la (E)-4,4-diméthyl-2-heptadéc-8-ényl-1,3-oxazoline, la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline. De manière encore plus avantageuse, ladite oxazoline est la 2-undécyl-4,4-diméthyl-1,3-oxazoline, appelée OX-100 ou Cyclocéramide®.

Les agents hypopigmentants pouvant être utilisés en association sont l'hydroquinone et ses dérivés, l'arbutine, l'acide rétinoïque, le rétinol, le rétinaldéhyde, l'acide kojique, l'acide azélaïque, la vitamine B3 ou PP, les dérivés du résorcinol, le résvératrol, des extraits de licorice ou de murier blanc, l'acide alpha-lipoïque, l'acide linoléique, des chélateurs de cations tels que l'EDTA (acide éthylène diamine tétra acétique), les extraits de soja. On peut également citer le Sepiwhite® (N-undecylenoyl-L-phénylalanine) commercialisé par la société Seppic, qui est un agent cosmétique dépigmentant.

Comme exemple d'agents pigmentants, on peut notamment citer
- les agents qui colorent la peau : le dihydroxyacétone, les mélanines ;
- les agents qui stimulent le procédé de pigmentation naturelle : les psolarènes (8-méthoxypsolarène, 5-méthoxypsolarène, 4,5',8-triméthylpsolarène ou des extraits végétaux de *Psorelea corylifolia* et de *Ammi majus*), les caroténoïdes (lycopène, canthaxanthine), les agents stimulant la voie de l'AMP cyclique (1. les analogues de l'AMPc, tels que le 8-bromo-AMPc ou le dibutiryl-AMPc, 2. la forskoline, 3. l'isobutyl-méthyl-xanthine ou la théophylline), les activateurs des protéines kinase C (diacylglycérols, en particulier oléyl-acétyl-glycérol), diols aliphatiques ou cycliques (1,2-propandiol, 5-norbomane-2,2-diméthanol, norbomane-2,2-diméthano), les diols bicycliques monoterpène, les dérivés de tyrosine (L-tyrosine, L-DOPA), le diméthylsulfoxyde, les agents lysomotropiques, les dinucléotides thymidine, les fragments d'ADN, les analogues de l'hormone stimulant les mélanocytes, le 3-isobutyl-1-méthylxanthine, les donneurs d'acide nitrique (Brown, Journal of photochemistry and photobiology B :biology 63 (2001) 148-161) ;
- les extraits végétaux, en particulier les algues, démontrant une activité promélanogène : *Laminaria digitata (*Thalitan de Codif).

Les extraits naturels de plante pouvant être utilisés en association sont avantageusement des extraits d'avocat, de lupin, de soja ou de tournesol , de mais et de colza voire de maca. On peut notamment citer les sucres d'avocat (Cf. la demande internationale WO2005/115421) ou les peptides d'avocat (Cf. la demande internationale WO2005/105123).

Les composés contenant des insaponifiables d'huiles végétales pouvant être utilisés en association sont avantageusement choisis dans le groupe constitué par les lipides furaniques d'avocat, les insaponifiables d'avocat et de soja, les concentrats d'huile de lupin, les concentrats d'huile de tournesol ,de maïs et de colza et leurs mélanges.

Les lipides furaniques d'avocat pouvant être utilisés en association sont avantageusement des 2-alkyl furanes naturels, notamment l'actif Avocadofurane® commercialisé par les Laboratoires Expanscience, pouvant être obtenus par le procédé décrit dans la demande internationale WO 01/21605.

Les insaponifiables d'avocat et de soja pouvant être utilisés en association sont avantageusement un mélange d'insaponifiables d'avocat furanique et d'insaponifiables de soja, dans un rapport respectif d'environ 1/3-2/3. Les insaponifiables d'avocat et de soja sont encore plus avantageusement le produit Piasclédine®, commercialisé par les Laboratoires Expanscience.

Les concentrats d'huile de lupin pouvant être utilisés en association sont avantageusement des concentrats obtenus par distillation moléculaire d'huile de lupin, avantageusement d'huile de lupin blanc doux , tels que ceux décrits dans la demande internationale WO 98/47479. Ils contiennent avantageusement environ 60% en poids d'insaponifiables.

Les concentrats d'huile de tournesol pouvant être utilisés en association sont avantageusement des concentrats de tournesol linoléiques, tels que l'actif commercialisé par les Laboratoires Expanscience, Soline® (cf. la demande internationale WO 01/21150).

Les insaponifiables « stéroliques » sont des insaponifiables dont la teneur en stérols, en méthylstérols et en alcools triterpèniques est comprise entre 20 et 95 % en poids, de préférence 45-65 % en poids, par rapport au poids total de l'insaponifiable.

Les plantes hypoglycémiantes pouvant être utilisés en association sont avantageusement choisis dans le groupe constitué par Le fenugrec (*Trigonella graenum*), l'acide corosolique (composé actif des feuilles de l'arbre *Lagestroemia speciosa*), le *Gymnema syllvestre,* le jus de fruit de momordique (*Momormodica charantia*), l'eucalyptus (*Eucalyptus globulus*), le *Panax ginseng,* les feuilles de myrtille (*Vaccinum myrtillus*).

Les oligo-éléments pouvant être utilisés en association sont avantageusement choisis dans le groupe constitué par le magnésium, le chrome, le sélénium et leurs mélanges.

La composition selon l'invention peut être formulée sous la forme de différentes préparations adaptées à une administration topique, à une administration orale, rectale, vaginale, nasale, auriculaire ou bronchique, à une administration parentérale.

Selon une première variante, les différentes préparations sont adaptées à l'administration topique et incluent les crèmes, les émulsions, les laits, les pommades, les lotions, les huiles, les solutions aqueuses ou hydro -alcooliques ou glycoliques, les poudres, les patchs, les sprays ou tout autres produits pour application externe.

Selon une deuxième variante, les différentes préparations sont adaptées à une administration orale ; l'extrait de quinoa pouvant entrer soit dans une composition alimentaire soit dans un complément alimentaire. Le complément alimentaire peut se présenter sous forme de l'extrait de quinoa en tant que tel (par exemple huile raffinée éventuellement enrichie en sa fraction insaponifiable) ou bien sous forme de gélules ou de capsules molles de gélatine ou végétales dans le cadre de la présente invention. Ledit complément alimentaire peut alors contenir de 10 à 100% en poids de l'extrait de quinoa.

Selon cette deuxième variante de la présente invention, on peut incorporer, sans aucune restriction, les extraits de quinoa de la présente invention dans la nourriture, les boisons et les nutraceutiques, y compris dans ceux cités ci-dessous:
1) Les produits laitiers : tels que les fromages, le beurre, le lait et autres breuvages lactés, mélanges et pâtes à tartiner à base de produits lactés, crèmes glacées et yaourts ;
2) Les produits à base de graisse tels que les margarines, pâtes à tartiner, mayonnaises, matières grasses pour cuisson, huiles à frire et vinaigrettes ;
3) Les produits à base de céréales composés de graines tels que le pain et les pâtes, que ces aliments soient cuisinés, cuits au four ou transformés.
4) Les confiseries tels que le chocolat, les bonbons, les chewing gums, les desserts, les nappages, les sorbets, les glaçages, et autres garnitures ;
5) Les boissons alcoolisées ou non, y compris les sodas et autres boissons non alcoolisées, jus de fruits, compléments diététiques, substituts de repas sous forme de breuvage comme ceux vendus sous la marque Boost™ and Ensure™ et ;
6) Les produits divers comme les oeufs, les aliments transformés comme les soupes, les sauces toute prête pour pâtes, des plats préparés et autre produits du même genre.

La composition de la présente invention peut être incorporée directement et sans autre modification dans la nourriture, les nutraceutiques, les produits diététiques notamment hyperprotéinés ou les breuvages et ce grâce à des techniques comme le mixage, l'infusion, l'injection, le mélange, l'absorption, le pétrissage et la pulvérisation.

Les modes d'administration, les posologies et les formes galéniques optimales des composés et compositions selon l'invention peuvent être déterminés selon les critères généralement pris en compte dans l'établissement d'un traitement pharmaceutique, en particulier dermatologique, ou vétérinaire adapté à un patient ou à un animal comme par exemple l'âge ou le poids corporel du patient ou de l'animal, la gravité de son état général, la tolérance au traitement, les effets secondaires constatés, le type de peau. En fonction du type d'administration souhaitée, la composition et/ou les composés actifs selon l'invention peuvent en outre comprendre au moins un excipient pharmaceutiquement acceptable, notamment dermatologiquement acceptable. Selon la première variante, on utilise un excipient adapté pour une administration par voie topique externe. La composition selon la présente invention peut en outre comprendre au moins un adjuvant pharmaceutiquement connu de l'homme du métier, choisi parmi les épaississants, les conservateurs, les parfums, les colorants, des filtres chimiques ou minéraux, les agents hydratants, les eaux thermales, etc.

La composition comprenant une huile raffinée de quinoa ayant les spécifications indiquées est particulièrement destinée à une utilisation cosmétique, dermatologique ou alimentaire. Dans le cadre d'une utilisation cosmétique ou dermatologique, la composition sera avantageusement formulée sous la forme d'une préparation adaptée à une administration topique. Dans le cadre d'une utilisation alimentaire, à visée nutritive ou cosmétique (« cosmet-food), la composition sera avantageusement formulée sous la forme d'une préparation adaptée à une administration orale. Elle pourra ne pas comprendre d'excipient et être constituée, en intégralité, de l'huile de quinoa raffinée.

La composition comprenant une huile raffinée de quinoa enrichie en sa fraction insaponifiable est particulièrement destinée à une utilisation cosmétique, dermatologique ou alimentaire. Dans le cadre d'une utilisation cosmétique ou dermatologique, la composition sera avantageusement formulée sous la forme d'une préparation adaptée à une administration topique. Dans le cadre d'une utilisation alimentaire, à visée nutritive ou cosmétique (« cosmet-food), la composition sera avantageusement formulée sous la forme d'une préparation adaptée à une administration orale. Elle pourra ne pas comprendre d'excipient et être constituée, en intégralité, de l'huile de quinoa raffinée concentrée en sa fraction insaponifiable.

La composition comprenant un insaponifiable est particulièrement destinée à une utilisation cosmétique ou dermatologique. La composition sera avantageusement formulée sous la forme d'une préparation adaptée à une administration topique.

La composition comprenant un extrait peptidique est particulièrement destinée à une utilisation cosmétique ou dermatologique. La composition sera avantageusement formulée sous la forme d'une préparation adaptée à une administration topique.

Il est également divulgué l'utilisation d'un extrait de quinoa, choisi parmi un extrait peptidique et osidique de quinoa ou un extrait lipidique de quinoa, ledit extrait lipidique de quinoa étant lui-même choisi dans le groupe constitué par une huile concentrée en sa fraction insaponifiable, un insaponifiable ou une huile raffinée ayant les spécifications données dans le tableau 2, pour la fabrication d'une composition dermatologique ou d'un aliment fonctionnel.

Un aliment fonctionnel est un aliment conventionnel, ou qui en a l'apparence, qui fait partie de l'alimentation normale, et qui a pour caractéristique de procurer des effets physiologiques bénéfiques dépassant ses fonctions nutritionnelles habituelles ou de réduire le risque de maladies chroniques.

Il est également divulgué une methode de traitement cosmétique, de soin hygiénique, d'embellissement et/ou une méthode pour parfumer des muqueuses et/ou des peaux normales, sèches, grasses, mixtes, déshydratées, âgées, sensibles, irritées, inconfortables, intolérantes, présentant un déséquilibre lié au vieillissement intrinsèque, extrinsèque ou hormonal ou lié aux agressions exogènes (polluants, UV, stress...), à tendance allergique, présentant des troubles de la pigmentation, présentant un aspect disgracieux lié à la surcharge de la masse graisseuse, caractérisée en ce qu'elle consiste à administrer une composition ou un aliment fonctionnel selon l'invention.

Il est concerne par ailleurs divulgué une méthode de traitement des phanères (cheveux, poils, ongles) caractérisée en ce qu'elle consiste à administrer une composition ou un aliment fonctionnel selon l'invention.

En particulier, la composition ou l'aliment fonctionnel est destiné à la prévention et au traitement des réactions ou pathologies allergiques, inflammatoires, irritatives ou des troubles de la barrière ou de l'homéostasie
- de la peau, telles que l'acné, la dermatite atopique, la dermite séborrhéïque, la rosacée, le psoriasis, les troubles vasculaires, la dermite du siège, les dartres, les gerçures, les piqûres, les crevasses en particulier des seins, les coups de soleil, les inflammations dus aux rayons de toutes sortes, les irritations ou allergies (par agents chimiques, physiques (contrainte de tension : femmes enceintes), bactériologiques, fongiques ou viraux, parasitaires (poux, gale, teigne, acariens, dermatophytes), radiologiques ou de rayonnement (UV, IR) ou par déficit de l'immunité innée (peptides antimicrobiens) ou acquise (cellulaire,humorales,cytokines)), les vergetures et/ou
- des muqueuses (gengivites (sensibles du nouveaux nés, d'hygiène, dues au tabagisme), parodontopathie, les irritations des sphères génitales males ou femelles externes ou internes) et/ou
- des phanères (alopécie, pellicules, hirsutismes, dermites séborrhéiques) immatures, normales ou matures.

La composition ou l'aliment fonctionnel peut également être destiné à la régénération tissulaire et pour favoriser la cicatrisation, ou peut être également destiné à protéger et renforcer la barrière cutanée, à réguler les troubles de la pigmentation et à agir sur les mécanismes de lipolyse et de lipogénèse.

L'huile raffinée de quinoa, éventuellement concentrée en sa fraction insaponifiable, présente en outre les avantages suivants : elle permet de diminuer le risque d'athérogénèse, elle présente des propriétés hypocholestérolémiantes, elle agit dans la prévention de certains cancers et des maladies cardio-vasculaires, dans la stimulation de la réponse immunitaire chez les personnes âgées, dans la réduction du risque de cataracte et dans le retard de la progression des maladies neurovégétatives.

Un autre avantage de l'huile raffinée de quinoa, éventuellement concentrée en sa fraction insaponifiable, est qu'elle peut être utilisée en cosmétique (« food-cosmétique »), plus particulièrement en vue d'améliorer l'aspect cutané, pour hydrater la peau, maintenir en l'état la barrière cutanée et le ciment intercornéocytaire par un apport en acides gras essentiels et en stérols, en vue de prévenir le vieillissement cutané par piégeage des radicaux libres, et en tant qu'agent anti-inflammatoire ou protectrice solaire.

Selon une variante préférée de l'invention, l'huile raffinée de quinoa, concentrée en sa fraction insaponifiable, est utilisée dans le traitement des troubles liés au tissu dermique. Le tissu conjonctif dermique joue un rôle majeur en tant que support et soutien au niveau de la peau, absorbeur de choc, le derme est notamment responsable de la fermeté et de la souplesse. La dégénérescence de ce tissu, associée à une altération du réseau collagénique (collagènes, en particulier de type I, III, II et V) ou élastique (élastine - inhibition de la synthèse, synthèse imparfaite, dégradation des fibres de collagène diminution du nombre des fibroblastes est de leur métabolisme...) peuvent donc avoir des conséquences importantes sur :
- le vieillissement cutané (chronologique, extrinsèque ou photo-vieillissement et ménopausique), notamment caractérisé par une diminution du nombre et de l'activité des fibroblastes, ainsi qu'une dégradation excessive de la matrice extracellulaire ;
- les vergetures, atteinte de la cellule fibroblastique caractérisée par une inflammation, une inhibition de l'expression des gènes codant pour la fibronectine, les collagènes de type I et III et de l'élastine, une transformation des fibroblastes en myofibroblastes sous l'effet des distensions mécaniques. Cette dégénérescence du tissu collagénique conduit à la formation d'une cicatrice dermique atrophique. Les principaux facteurs déclenchant sont : l'inflammation et le stress mécanique et l'environnement hormonal (lors de la grossesse). Les vergetures atteignent près de 50% de la population jeune essentiellement féminine. Elles s'observent généralement au cours de la grossesse (60 à 70% des femmes enceintes), au cours de la puberté (25% des filles pour 10% de garçon), ou au cours de certaines maladies (métaboliques, endocriniennes et infectieuses). Ce sont des lésions linéaires, légèrement déprimées, étroites, orientées dans le sens des lignes de tensions cutanées et recouvertes d'un épiderme plissé. Leur couleur varie selon le stade évolutif: elles ont une couleur rouge, voie violine au début, puis prennent un aspect blanchâtre nacré dans un second temps :
- les plaies profondes atteignant le derme, elles provoquent une altération du tissu dermique avec une diminution du nombre des fibroblastes et une dégradation de la matrice. Le mécanisme de cicatrisation se met en place pour réparer le tissu altéré : les fibroblastes prolifèrent et la matrice extracellulaire est remodelée : synthèse des différents composants.

Il est divulgué l'utilisation de ladite huile enrichie en insaponifiables pour la prévention et/ou le traitement du vieillissement cutané, des vergetures et des plaies profondes. Ladite huile enrichie en insaponifiables peut également servir pour favoriser la cicatrisation.

Selon une autre variante avantageuse de l'invention, ladite huile enrichie en insaponifiables peut être utilisée dans la prévention et/ou le traitement des atrophies sous - cutanées du derme. Les atrophies sous-cutanées sont un problème fréquemment rencontré en dermatologie. Elles peuvent être secondaires à différentes étiologies. Selon leur localisation, ces lésions représentent une gêne esthétique mineure ou au contraire handicapent fortement la personne

Les atrophies sous-cutanées peuvent avoir différentes étiologies. Au premier rang figurent les atrophies cicatricielles soit post-traumatiques (traumatismes jusqu'au derme), soit post-inflammatoires (par exemple post-acné). Les cicatrices atrophiques post-traumatiques comportent une atrophie épithéliale avec une membrane basale linéaire témoignant du remaniement de la jonction dermo-épidermique avec une perte du dessin papillaire. Histologiquement, l'épaisseur du derme est diminuée, les fibres de collagène sont ténues et les fibrocytes souvent plus nombreux que dans une peau normale. L'atrophie dermique comporte aussi une hypotrophie des annexes pilosébacées et parfois sudorales. Les cicatrices issues d'un processus inflammatoire se constituent plus souvent dans le derme profond et l'hypoderme. Il y a un épaississement du derme qui s'accompagne d'une sclérose. Les constituants de la matrice extracellulaire sont progressivement remplacés par des fibres de collagène épaissies et denses. Ce processus de sclérose s'accompagne d'une diminution de la vascularisation dermique et des annexes. On parle à ce stade de scléro-atrophie, un état que l'on peut observer dans une sclérodermie localisée (morphée). Ce processus peut aussi toucher l'hypoderme dans un contexte inflammatoire immunitaire (lupus profond, syndrome de Perry-Romberg), médicamenteux (trithérapie, injection de corticoïdes), enzymatiques (cytostéatonécroses pancréatiques), ou traumatique (atrophie de l'hypoderme chez des femmes portant des bas à mi-cuisses).

D'autres atrophies sont répertoriées : consécutive à un traitement local aux dermocorticoïdes, consécutive à la ménopause et en association ou non avec le THS (traitement hormonal substitutif), dues à certaines maladies génétiques ou non, hypoplasie, maladie du tissu conjonctif de la peau du collagène, syndrome de goltz, atrophodermie de Pasini et Pierini , keratose pilaire atrophiante. Enfin lors des greffes de peau, des brûlures, des pertes de substances cutanée de toutes origines, des escarres.

Il est donc proposé de combler l'atrophie du derme par un traitement à base de concentrât (= huile enrichie en insaponifiables) de quinoa qui relance l'activité protéique.

En particulier, la présente invention a pour objet une composition comprenant une huile concentrée en sa fraction insaponifiable, ayant les spécifications données précédemment (tableau 3) pour son utilisation :
- dans la prévention et/ou le traitement des plaies profondes,
- pour favoriser la cicatrisation, ou
- dans la prévention et/ou le traitement des atrophies sous-cutanées du derme.

La présente invention a également pour objet une méthode de traitement cosmétique visant à prévenir ou à traiter le vieillissement cutané ou les vergetures en utilisant la composition comprenant une huile concentrée en sa fraction insaponifiable, ayant les spécifications données précédemment (tableau 3).

La présente invention a également pour objet une composition comprenant un extrait peptidique et osidique de graines de quinoa, susceptible d'être obtenu par un procédé comprenant les étapes successives suivantes :
a) à partir de graines de quinoa, extraction d'une huile brute et d'un tourteau et récupération dudit tourteau ;
b) lavage dudit tourteau par l'eau ou un mélange hydroalcoolique pour ne conserver que la partie protéique, puis
c) solubilisation des protéines ;
d) concentration des protéines puis hydrolyse desdites protéines en peptides;
e) purification et récupération de l'extrait peptidique et osidique,
et le cas échéant un excipient approprié,
pour son utilisation :
- pour favoriser la cicatrisation;
- dans la prévention et/ou le traitement de l'acné ;
- pour réguler les troubles de la pigmentation ; ou
- dans la prévention et/ou le traitement des hirsutismes ou des dermites séborrhéiques.

L'invention a également pour objet une méthode de traitement cosmétique des phanères, en particulier des cheveux, des poils, ou des ongles, de l'alopécie, des pellicules, ou des peaux normales, sèches, grasses, mixtes, déshydratées, âgées ou sensibles, consistant à administrer une composition comprenant un extrait peptidique et osidique de graines de quinoa, susceptible d'être obtenu par un procédé comprenant les étapes successives suivantes :
a) à partir de graines de quinoa, extraction d'une huile brute et d'un tourteau et récupération dudit tourteau ;
b) lavage dudit tourteau par l'eau ou un mélange hydroalcoolique pour ne conserver que la partie protéique, puis
c) solubilisation des protéines ;
d) concentration des protéines puis hydrolyse desdites protéines en peptides ;
e) purification et récupération de l'extrait peptidique et osidique.

Avantageusement, l'extrait peptidique et osidique est constitué de 25 à 90% en poids de peptides et de 10 à 50% en poids de sucres, les pourcentages étant exprimés en poids par rapport au poids total dudit extrait peptidique et osidique.

Selon une variante préférée de l'invention, l'extrait peptidique et osidique de quinoa est utilisé dans la cicatrisation épidermique.

Une perturbation de l'intégrité de la peau peut survenir dans plusieurs contextes. La peau peut subir des dommages lors de chirurgies, de brûlures, de radiations, de coupures, d'éraflures, de frottements, et de pressions. Le degré de gravité de la blessure varie selon certains facteurs comme l'étendue, la profondeur et la nature. Afin de maintenir les fonctions essentielles de la peau, il est très important de la réparer lorsqu'un tel événement survient. La guérison d'une plaie cutanée représente l'ensemble des processus qui mènent à la fermeture de la plaie et à la récupération fonctionnelle du tissu cutané. L'épiderme guérit par régénération ou ré-épithélialisation, c.-à-d. qu'il récupère sa structure et ses fonctions originales. Incapable de répondre à une lésion par la régénération, le derme guérit par réparation, c.-à-d. que le tissu d'origine est remplacé par un tissu conjonctif non spécifique avec, comme résultat, la formation d'une cicatrice moins fonctionnelle (ex. résistance mécanique inférieure). Ces processus impliquent des populations cellulaires différentes, des compartiments cellulaires distincts (épiderme et derme), divers médiateurs et des interactions multiples entre tous ces éléments, le tout variant en fonction du temps.

La ré-épithélialisation consiste en la régénération par les kératinocytes d'un épithélium organisé, pavimenteux, stratifié, kératinisé, qui recouvre la plaie et qui reforme une barrière protectrice contre l'environnement extérieur afin de réduire la mortalité à la suite d'une blessure. Le mécanisme de ré-epithélialisation se fait selon 3 étapes qui se déroulent en parallèle mais de manière décalée dans le temps : (1) migration des kératinocytes (la migration cellulaire peut être influencée par plusieurs mécanismes tel que la perte d'inhibition de contact; la présence de médiateurs inflammatoires comme les facteurs de croissance, ou des protéines sécrétées par les cellules, mais également par les différents contacts avec des substrats de la matrice comme la fibronectine et la Laminine 5); (2) prolifération cellulaire (une vague mitotique se produit pour combler l'espace laissé par les cellules migrantes et pour recouvrir la lésion. La prolifération des kératinocytes qui se produit après 48 à 72 heures, ne semble pas affecter la migration. Elle s'effectue sous l'influence de nombreux facteurs qui peuvent être sécrétés par les cellules avoisinantes comme les fibroblastes ou par les kératinocytes eux-mêmes : KGF (Kératinocyte growth Factor), IL-1, IL-6, IL-8, Colony Stimulating Factor (CSF), PDGF, TNF-a, IGF-1 (Insulinase Growth Factor)), (3) maturation de l'épiderme (la maturation et la différenciation de l'épiderme se font simultanément avec la fermeture de la plaie et correspondent à une reprise de la fonction et de la morphologie normale des kératinocytes.). Les kératinocytes s'activent, adaptent leur morphologie à la migration, migrent et prolifèrent sous l'influence de différents facteurs de croissance pour ré-épithélialiser la plaie. Avec l'avancement du recouvrement de la plaie, le néo-épiderme débute sa maturation afin de reformer une couche cornée protectrice.

Certains facteurs de croissance, qui contrôlent la migration des kératinocytes, sont également capables d'influencer la migration. C'est le cas de l'EGF et du TGF-β qui la stimulent en augmentant l'expression de l'intégrine α2β1 à la surface des kératinocytes mais aussi du TGFβ qui est l'un des facteurs majeurs impliqués dans la migration et qui agit en activant la synthèse matricielle.

L'interaction cellule-matrice est importante au cours de la cicatrisation de la plaie. En effet, la matrice extracellulaire contient des substances adhésives et des fibres qui guident les cellules migrantes. De la même façon, des molécules présentes dans le sang peuvent contribuer à la migration cellulaire. Par exemple, la fibrine et la fibronectine, s'attachent à la matrice provisoire et forment une structure sur laquelle les kératinocytes peuvent migrer. Les kératinocytes en migration élaborent également des éléments de cette matrice. Les kératinocytes synthétisent la laminine 5, le collagène V et l'antigène de la pemphigoïde bulleuse. L'effet des divers substrats sur la migration des kératinocytes est médié par des intégrines et la sécrétion de protéases de la matrice extra-celulaire (MMP-1, MMP-2 et MMP-9), qui leur permettent successivement de s'accrocher et de se libérer de ces substrats.

La laminine 5 est une protéine spécifique des lames basales des épithéliums qui ont des fonctions de sécrétion ou de protection, comme les muqueuses ou la peau. La laminine 5 est considérée comme le composant clé du complexe d'ancrage de l'épiderme et comme étant la protéine contribuant le plus à la stabilité de la membrane basale. La laminine 5 résulte de l'assemblage hétérotrimérique de sous-unités α3, β3 et γ2 et est synthétisée exclusivement par les cellules épithéliales sous la forme d'un précurseur. Le rôle majeur de la laminine 5 est souligné par l'existence de maladies héréditaires ou acquises, résultant d'une anomalie de synthèse et/ou d'expression de l'une de ses sous-unités constitutives. Ces maladies, appelées épidermolyses bulleuses jonctionnelles, conduisent notamment à une fragilité de la jonction dermo-épidermique de la peau caractérisée par la formation spontanée de bulles épidermiques. Ainsi, la laminine 5 a un rôle biologique déterminant puisqu'elle permet l'adhérence des cellules épithéliales adjacentes. En plus de son rôle dans l'adhérence stable, la laminine 5 joue un rôle important au cours de la migration cellulaire puisqu'elle est fortement exprimée par les kératinocytes migrantes dans les phases précoces de la cicatrisation épidermique. Dans la peau normale, il n'y a peu ou pas de marquage de la laminine 5 dans le cytoplasme des kératinocytes basaux tandis que dans une plaie en guérison, la laminine 5 est détectée dans les cellules basales. La forme longue de la laminine 5 interagit avec les intégrines α3β1 et α2β1 qui sont deux récepteurs retrouvés dans les plaques d'adhésion focale utiles pour le mouvement cellulaire. L'intégrine α2β1 semble être impliquée de manière prépondérante dans la migration dess kératinocytee. Au cours de la migration, la régulation de l'expression de la laminine 5 est mediée par le TGF-β et l'INF-γ.

La cicatrisation cutanée est associée à des événements de migration et de remodelage de la matrice qui ont recourt à l'action des métalloprotéases matricielles (MMPs). Les kératinocytes se déplacent donc à travers une matrice temporaire qu'ils dégradent au besoin pour faciliter leur migration et dont ils vont progressivement modifier la composition. Les MMPs constituent une famille d'enzymes, zincdépendantes, de structure très conservée, et qui possèdent la capacité de dégrader les composants de la matrice extracellulaire. Elles peuvent être synthétisées par différents types cellulaires au niveau de la peau (fibroblastes, kératinocytes, macrophages, cellules endothéliales, éosinophiles, cellules de Langerhans,...). Le rôle prépondérant des MMPs dans le remodelage protéolytique de la matrice extracellulaire est maintenant clairement établi dans la cicatrisation cutanée. L'effet des divers substrats sur la migration des kératinocytes est médié par la sécrétion de protéases de la matrice extra-celulaire : MMP-1, MMP-2 et MMP-9, qui leur permettent successivement de s'accrocher et de se libérer de ces substrats. La MMP-9 est exprimée de manière prépondérante au cours de la cicatrisation cutanée et intervient dans la phase de migration et de remodelage de la matrice. Cette protéase serait également un élément majeur d'une cicatrisation sans cicatrice.

Or, il a été montré que l'extrait pour favoriser la cicatrisation selon l'invention agit directement sur les 2 premières étapes impliquées dans la ré-épithélialisation (migration et prolifération cellulaire).
- Stimulation de la migration des kératinocytes :
   ○ Action sur l'expression des gènes codant pour les 3 chaînes composant la laminine 5 (α3β3γ2) ;
   ○ Action sur la synthèse de la MMP-9 via une augmentation de l'expression de son gène ;
   ○ Action sur la migration des kératinocytes
- Stimulation de la Prolifération des kératinocytes :
   ○ Action directe sur la prolifération cellulaire
   ○ Action indirecte des fibroblastes : sécrétion de plus de KGF: facteur de croissance activant la division cellulaire des kératinocytes

Ainsi, l'invention a pour objet l'utilisation d'un extrait peptidique et osidique de quinoa, tel que décrit précédemment, pour favoriser la cicatrisation. En particulier, ledit extrait peptidique et osidique peut être utilisé pour la prévention et/ou le traitement des cicatrices superficielles telles que : les cicatrices post-acné, les cicatrices post-peeling, les cicatrices post-laser, les cicatrices post-brûlures et les éraflures. Ledit extrait peptidique et osidique peut également être utilisé en tant que soin (cosmétique) pour les lèvres fragiles et les cheilites. Ledit extrait peptidique et osidique peut aussi être utilisé dans la prévention du vieillissement cutané, en raison d'un défaut de cicatrisation avec l'âge. Cet extrait peptidique et osidique peut également être utilisé dans le traitement et/ou la prévention des vergetures.

Cet extrait peptidique et osidique peut aussi être utilisé pour réparer la peau après piqûres (moustiques). Il permet également une réparation des abrasions de la peau par mécanismes physiques (grattage, prurit, frottement mécanique, rayonnement laser), chimiques et biochimiques (peeling, érythème fessier par exemple). En particulier, cet extrait peut être utilisé dans le traitement cosmétique des boutons et/ou des croûtes, permettant une réparation de la peau après des boutons dus à des pathologies telles que l'acné ou les varicelles, et/ou des croûtes dues à des pathologiques (atopie, croûtes de lait).

Cet extrait peptidique et osidique trouve également une application dans le traitement cosmétique des peaux fragiles et sensibles.

Les exemples qui suivent illustrent l'invention mais ne sont pas limitatifs.

### Exemple 1: huile raffinée de quinoa (désodorisée) : procédé de préparation et spécifications

L'huile raffinée de quinoa est obtenue par extraction au solvant (n-hexane) des graines de quinoa (1000kg, rendement de 5,5%) et obtention de l'huile brute de quinoa (55kg). Cette huile brute est ensuite raffinée (rendement de 70%) pour conduire à l'huile de quinoa raffinée (38,5kg). On obtient une huile de couleur jaune trouble avec dépôt ; on ne retrouve pas de traces d'hexane.

L'huile raffinée de quinoa désodorisée présente les spécifications suivantes : Indice de peroxyde : 7,3 meq/kg ; Indice d'acide : 0,30 mg KOH/g
Composition en acides gras : C14 0,1%; C16 8,2% ; C16' 0,2% ; C18 0,8%, C18' 30,4%, C18" 47,2% ; C18"' 8,3% ; C20 0,6% ; C20' 1,7%, C22 0,7% ; C22' 1,6%; C24 0,2%.
Teneur en tocophérols totaux : 4,8 mg/100g
   % relatif α-tocophérol: 22,3% ; % relatif β-tocophérol : 0,0% ; % relatif
   γ-tocophérol: 61,5% ; % relatif δ-tocophérol : 16,2%
Teneur en stérols totaux : 1,63 g/100g
   % relatif campestérol: 1,53% ; % relatif stigmastérol : 3,19% ; % relatif
   β-sitostérol : 20,00% ; % relatif δ-5-avenastérol: 1,7%; % relatif
   δ-7-stigmastérol: 46,35% ; % relatif δ-7-avenastérol: 8,55%
Teneur en squalène : 2,5 g/100g

### Exemple 2: huile raffinée de quinoa concentrée en sa fraction insaponifiable (=concentrât d'huile de quinoa) : procédé de préparation et spécifications

L'huile de quinoa raffinée (désodorisée, 38,5 kg) est soumise à une étape de distillation moléculaire pour conduire à une huile raffinée de quinoa concentrée en sa fraction insaponifiable, encore dénommée concentrât d'huile de quinoa (3,85kg, rendement de 10%).

L'huile raffinée de quinoa désodorisée concentrée en sa fraction insaponifiable présente les spécifications suivantes :
Huile de couleur jaune trouble avec dépôt ; on ne retrouve pas de traces d'hexane Indice de peroxyde : 1,43 meq/kg ; Indice d'acide : 3,04 mg KOH/g
Composition en acides gras : C14 0,3% ; C16 12,4% ; C16' 0,3% ; C18 0,7% ; C18' 29,4% ; C18" 47,1% ; C18"' 7,7% ; C20 0,3% ; C20' 0,9% ; C22 0,3% ; C22' 0,6% ; C24 0,1%.
Teneur en tocophérols totaux : 58,7 mg/100g
   % relatif α-tocophérol: 72,3% ; % relatif β-tocophérol: 0,7% ; % relatif
   γ-tocophérol : 23,1%; % relatif δ-tocophérol : 4,0% ;
Teneur en stérols libres: 0,4 g/100g
Teneur en stérols totaux: 7,73 g/100g
   % relatif campestérol : 5,57% ; % relatif stigmastérol : 3,4% ; % relatif
   β-sitostérol : 26,84% ; % relatif δ-5-avenastérol: 2,3% ; % relatif
   δ-7-stigmastérol: 39,48% ; % relatif δ-7-avenastérol : 5,97%
Teneur en squalène : 16,8 g/100g

### Exemple 3: extrait peptidique et osidique de quinoa : procédé de préparation et spécifications

Un extrait peptidique et osidique de quinoa a été préparé suivant le protocole suivant :

Matière première de départ : tourteau de quinoa, à 10-12% de protéines en poids par rapport au poids de la matière sèche (MS= matière sèche). Ce tourteau de quinoa est soumis à une extraction alcaline (ajustement du pH à pH 10). Le surnageant est ensuite soumis à une ultrafiltration à l'aide de membranes minérales 8 kDa (enrichissement en protéines). Ensuite, le rétentat est soumis à une étape de concentration avant l'étape d'hydrolyse enzymatique. L'hydrolyse enzymatique des protéines est réalisée avec la Prolyve 1000, à une température de 55°C, à un pH de 8. A la fin de l'étape d'hydrolyse enzymatique, l'enzyme est désactivée par un traitement thermique. L'hydrolysat est ensuite soumis à une étape d'ultrafiltration à l'aide de membranes minérales 8 kDa (récupération des peptides dans le filtrat). L'ultrafiltrat est concentré, puis éventuellement soumis à une filtration stérilisante sur 0,2 µm et/ou lyophilisé.

L'extrait peptidique et osidique de quinoa présente les spécifications suivantes : Poudre de couleur jaune orangée sans conservateurs

### Composition par rapport à la poudre (%, p/p)

Azote α-aminé (OPA, équivalent leucine) : 13% ± 20%
Protéines (Biuret, équivalent BSA) : 27 % ± 20%
Sucres totaux (anthrone, équivalent glucose) : 24% ± 20%

**Tableau 5 : profil de répartition des masses molaires des peptides et acides aminés**

| | | | | | |
|---|---|---|---|---|---|
| Masse molaire (Da) | > 3500 | 3500 - 1200 | 1200 - 300 | 300 - 130 | < 130 |

| Répartition des peptides (%) | | | | | |
|---|---|---|---|---|---|
| | 2 | 15,5 | 44,5 | 15 | 23 |

### Exemple 4: huile raffinée de quinoa concentrée en sa fraction insaponifiable (=concentrât d'huile de quinoa) : activités biologiques

On a évolué l'effet du concentrât d'huile de Quinoa, obtenu à l'exemple 2, sur différents paramètres de la matrice dermique: (a) effet sur la prolifération des fibroblastes, (b) effet sur la matrice extracellulaire du derme : expression génique du Collagène I, Collagène III et de 1' Elastine, (c) effet sur la distension mécanique du derme : effet sur les forces isométriques développées par des fibroblastes issus de vergetures rouges.

### Matériel et Méthodes :

### a. Etude de la prolifération des fibroblastes dermiques

Le test au MTT [3-(4,5-dimethylthiazol-2-yl)-2,5-diphenyltetrazolium bromide] est un test colorimétrique qui mesure la viabilité cellulaire. Le MTT est un sel de tétrazolium hydrosoluble de couleur jaune ; les cellules métaboliquement actives sont capables de le réduire en cristaux de formazan bleus.

A J0, les fibroblastes sont ensemencés en milieu RPMI à 1% SVF en plaque 96 puits. A J1, les cellules sont traitées par le concentrât d'huile de Quinoa dans du milieu RPMI à 1% SVF à 0,005% et 0,01% de matière sèche (MS) ou par du milieu RPMI à 10% SVF (contrôle Positif) pendant 24 et 48 heures.

En fin de traitement, la viabilité cellulaire est quantifiée par un test au MTT : après 3 heures de contact avec le MTT, les cristaux de formazan formés sont solubilisés par du DMSO et la densité optique, proportionnelle à la quantité de cellules métaboliquement actives donc vivantes, est lue à 570 nm contre le blanc (puits sans cellules).

### b. Etude de l'effet des peptides de Quinoa sur l'expression des gènes codant pour le Collagène I et le collagène III et l'Elastine par RT-PCR

### b.1. Principe de la PCR Quantitative en temps réel :

La PCR Quantitative en temps réel ou QRT-PCR (pour Quantitative Real Time Polymerase Chain Reaction) est une méthode de biologie moléculaire, qui permet de mesurer de façon spécifique et quantitative l'expression de gènes d'intérêt par amplification. La quantification est basée sur le suivi de l'amplification des gènes en temps réel en utilisant comme système rapporteur la technologie SYBR Green : molécule aux propriétés fluorescentes qui s'intercale au sein de l'ADN double brin. La PCR se déroule en une succession de cycles de température selon 3 étapes :
- Dénaturation : séparation des 2 brins d'ADN.
- Hybridation : Reconnaissance d'une séquence d'ADN correspondant à un gène cible grâce aux amorces spécifiques.
- Extension : de la séquence d'intérêt par action d'une polymerase.

A la fin de la réaction, la quantification est réalisée en analysant le « cycle seuil » (Ct = point où le signal d'émission de fluorescence sera statistiquement et significativement plus élevé que le bruit de fond). Les quantités d'ADN sont comparées dans la partie exponentielle, moment pendant lequel l'augmentation de la quantité d'ADN est proportionnelle à la quantité initiale de matrice.

### b.2. Protocole :

A J0, les fibroblastes ont été ensemencés en plaques 6 puits dans du milieu RPMI additionné de 10% SVF. A J1, les fibroblastes ont été traités par le TGFβ1 à 5 ng/ml ou le concentrât d'huile de Quinoa à 0,005% et 0,01% MS dans du milieu RPMI à 1% SVF pendant 48 heures. A la fin du traitement des cellules, les ARN totaux ont été extraits (kit d'extraction RNeasy MiniKit ; Qiagen) puis dosés de façon quantitative en minichips à l'aide du système Experion (kit Experion RNA StdSens ; Biorad). Les ARN totaux ont ensuite été rétro-transcrits en cDNA (kit iScript cDNA Synthesis ; Biorad). Enfin, les cDNA néo-synthétisés relatifs aux gènes d'intérêt (Collagène I, Collagène III, Elastine) ou aux gènes de référence (HPRT, GAPDH, YWHAZ, beta actin = normalisateurs) ont été amplifiés sélectivement par PCR en temps réel (iQ5, Biorad) en utilisant des amorces spécifiques des séquences cibles.

L'expression des gènes de référence est analysée dans les mêmes échantillons que ceux pour lesquels l'expression des gènes d'intérêt est évaluée afin de normaliser les résultats et de s'assurer qu'ils sont bien le résultat de l'effet du traitement par le concentrât d'huile de Quinoa.

### b.3. Analyse de résultats :

Les résultats sont normalisés par rapport au gène de référence le plus stable (d'après l'algorithme geNorm) : DCt = Ct gène d'intérêt - Ct gène de référence le plus stable

La variation du nombre de copies du gène d'intérêt lors du traitement est ensuite calculée selon la formule suivante : DDCt = DCt contrôle - DCt traitement

Enfin, la quantité relative ou le niveau d'expression des gènes d'intérêt normalisé par le niveau d'expression des gènes de référence dans les échantillons non traités et traités est obtenue par la formule : QR= 2DDCt

### c. Evaluation de l'effet sur les forces isométriques développées par des fibroblastes issus de vergetures rouges

### c.1. Présentation du système GlaSbox®

L'inhibition mécanique de la rétraction du gel de collagène dans lequel les fibroblastes sont inclus se traduit par la génération d'une force, appelée « force de rétraction" ou "force isométrique".

Les lattices se développent dans une boite de culture qui est constituée de 8 cuves rectangulaires. Dans chacune d'elle plongent deux lames flexibles en silicium dont les parties inférieures sont constituées de grilles sur lesquelles s'accroche la lattice lors de sa polymérisation. La lattice se développe entre deux lames pour aboutir à une forme rectangulaire légèrement rétrécie au centre. Cette forme en mécanique classique est désignée comme une forme en diabolo. Ces lames sont équipées, au niveau de leur partie supérieure, d'un système de jauge de contrainte recouverte de fils d'or déposés à leur surface. Sous l'influence de la force de rétraction développée par les fibroblastes, les lames de silicium se déforment. Cela se traduit par une variation de la valeur de résistance électrique de la jauge de contrainte, mesurée par l'intermédiaire d'un pont de Wheatstone. Cette variation indique la force développée au sein de la lattice, mesurée en temps réel par le biais d'une carte d'acquisition PC et d'un logiciel adapté.

### c.2. Préparation des dermes équivalents sous tension et mesure des forces isométriques

Un milieu de fabrication des lattices est préparé en mélangeant : 6 volumes de milieu de culture avec 3 volumes de collagène I de queue de rat (2mg/ml) et un volume de suspension cellulaire (8,105 cellules/ml), le mélange est coulé dans les cuves rectangulaires de la GlaSbox. En quelques minutes à 37°C, un gel se constitue. Les différents milieux contenant ou non le principe actif sont ajoutés. Les forces isométriques seront mesurées durant 48 heures. A la fin de la manipulation, les lattices de collagène sont détachées et digérées dans une solution de collagénase. Après 2 heures d'incubation à 37°C, les cellules au sein de chaque lattice de collagène sont comptées. Les forces sont exprimées en fonction du nombre de cellule après 48 heures de manipulation. c.3. Analyse statistique : Les valeurs sont exprimées par la moyenne ± erreur type de la distribution des moyennes (sem). Une analyse de variance à 2 facteurs a été réalisée.

### Résultats

a. Prolifération des fibroblastes : Le traitement des fibroblastes par le concentrât d'huile de Quinoa à 0,005 et 0,01% MS, stimule de façon dose dépendante et significativement la prolifération (respectivement : +17 et +23% d'augmentation par rapport au contrôle sans traitement, après un traitement pendant 48H).

**Tableau 6: Etude de la prolifération des fibroblastes en présence du concentrât d'huile de Quinoa**

| | Traitement 24H | | | Traitement 48H | | |
|---|---|---|---|---|---|---|
| | Moyenne DO MTT | % augmentation | Student t test | Moyenne DO MTT | % augmentation | Studen t test |
| Contrôle | 0,754 | 100 | | 0,675 | 100 | |
| Contrôle Positif (RPMI-10%SVF) | 0,900 | 119 | p<0,01 | 1,249 | 185 | P<0,01 |
| Concentrât d'huile de Quinoa (0,005%) | 0,769 | 112 | p<0,05 | 0,753 | 117 | P<0,01 |
| Concentrât d'huile de Quinoa (0,01%) | 0,843 | 120 | p<0,01 | 0,793 | 123 | p<0,05 |

b. Expression du collagène I : L'analyse quantitative de la cinétique d'expression de l'ARNm du collagène I a été réalisée par PCR quantitative (Q-PCR) après 48 heures d'incubation avec le TGF-β1 à 5 ng/ml. Les résultats obtenus indiquent une induction importante de l'expression du gène collagène I (Tableau 7), Le concentrât d'huile de Quinoa stimule également de façon dose dépendante l'expression du collagène I (+58 et+67%).

**Tableau 7 : Etude de l'expression génique du collagène I**

| | Moyenne dCt | ddCt | QR | % d'induction |
|---|---|---|---|---|
| Contrôle | -2,3 | 0,00 | 1,00 | |
| TGFβ à 5 ng/ml | -3,2 | 0,9 | 1,87 | 87 |
| Concentrât d'huile de Quinoa 0,005% | 2,96 | 0,66 | 1,58 | 58 |
| Concentrât d'huile de Quinoa 0,01% | -3,04 | 0,74 | 1,67 | 67 |

c.Expression du collagène III : D'autre part, l'effet du concentrât d'huile de Quinoa sur l'expression du gène du collagène III a été analysé. Les résultats présentés dans le tableau 8, démontrent une augmentation importante de l'expression du gène collagène III (+92 et +62%).

**Tableau 8 : Etude de l'expression génique du collagène III**

| | Moyenne dCt | ddCt | QR | % d'induction |
|---|---|---|---|---|
| Contrôle | 4,92 | 0,00 | 1,00 | |
| TGFβ à 5 ng/ml | 2,51 | 2,4 | 5,27 | 427 |
| Concentrât d'huile de Quinoa 0,005% | 3,97 | 0,94 | 1,92 | 92 |
| Concentrât d'huile de Quinoa 0,01% | 4,22 | 0,69 | 1,61 | 62 |

d. Expression de l'Elastine : On a également mis en évidence un effet du concentrât d'huile de Quinoa sur l'expression du gène de l'élastine (tableau 9), avec une induction de +96 et +67% par rapport au contrôle sans traitement.

**Tableau 9 : Etude de l'expression génique de l'Elastine**

| | dCt | ddCt | QR | % d'induction |
|---|---|---|---|---|
| Contrôle | 4,58 | 0,00 | 1,00 | |
| TGFβ à 5 ng/ml | 2,16 | 2,4 | 5,35 | 435 |
| Concentrât d'huile de Quinoa 0,005% | 3,61 | 0,97 | 1,96 | 96 |
| Concentrât d'huile de Quinoa 0,01% | 3,84 | 0,74 | 1,67 | 67 |

e. Etude de l'effet du concentrât d'huile de Quinoa sur les forces contractiles développées par les fibroblastes de vergetures rouge au sein d'un derme equivalent sous tension dans le système GlaSbox®.

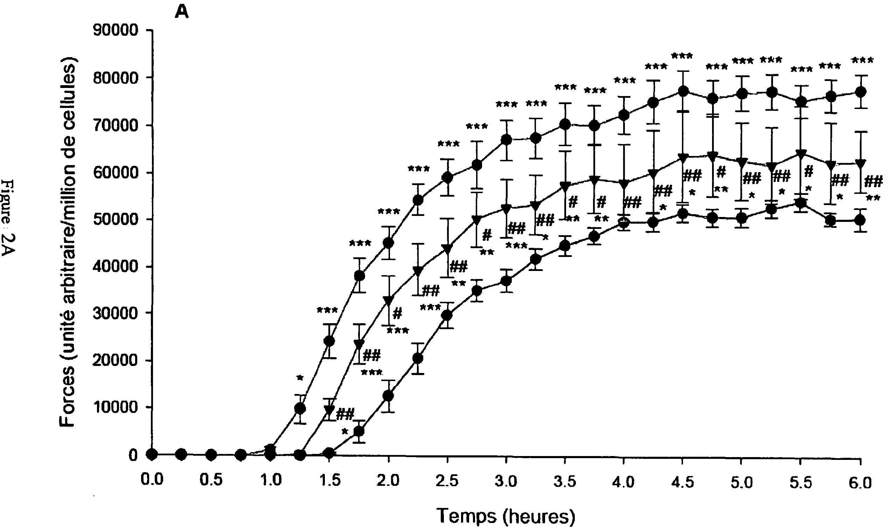

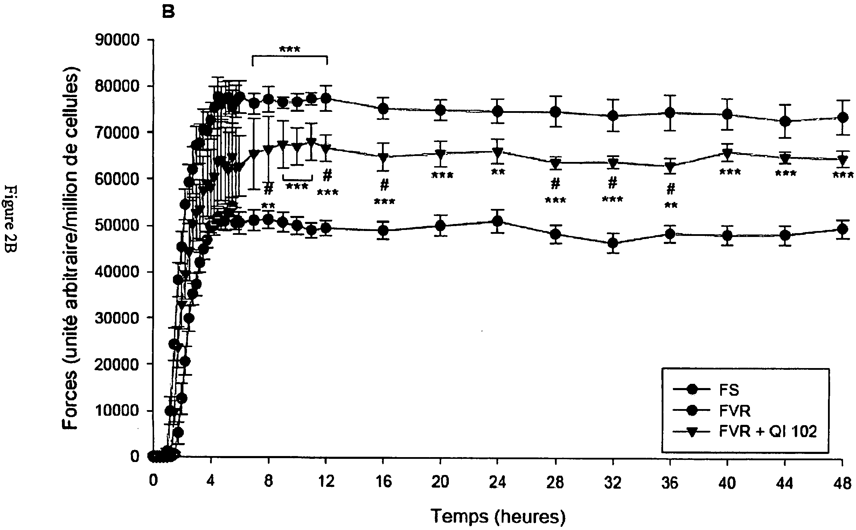

Comme montré figure 2, l'ajout de 0,01% de concentrât d'huile de Quinoa au milieu de culture diminue significativement les forces contractiles développées par les fibroblastes de vergetures rouges à partir de 1h30 de culture et ce jusqu'à la 36^{eme} heure. Les courbes obtenues lors de l'étude se composent de 3 phases distinctes :
- Phase I : la force isométrique reste faible pendant les deux premières heures de culture. Cette phase correspond à la polymérisation du gel de collagène.
- Phase II : la force isométrique augmente quasi linéairement jusqu'à un maximum, se situant en moyenne pendant les six à huit premières heures de culture. Cette phase correspond au temps nécessaire aux fibroblastes pour s'allonger et s'attacher aux fibres de collagènes.
- Phase III : la force isométrique se maintient au cours du temps de culture. Cette phase correspond au remaniement de la matrice de collagène par les fibroblastes et l'augmentation de l'expression de l'intégrine α2β1.

Le concentrât d'huile de Quinoa a un effet relaxant qui se maintient dans le temps car il diminue les forces isométriques développées par les fibroblastes issus de vergetures rouges à la fois durant la phase II et la phase III de la courbe.

Figure 2 : Forces contractiles développées au sein d'un derme équivalent sous tension dans le système GlaSbox® pendant 48 heures de culture (B) (A: détail des 6 premières heures) (moyenne ± sem) en présence ou en absence du concentrât d'huile de Quinoa (QI102). (*p<0,05 ; **p<0,01 et ***p<0,001 versus FS [Fibroblastes Saines] ; #p<0,05 et ##p<0,01 versus FVR [Fibroblastes de Vergetures Rouges])

### Conclusion

Les études présentées ici ont permit de mettre en évidence le rôle du concentrât d'huile de Quinoa sur la régulation du tissu dermique. En effet, il a été montré que le concentrât d'huile de Quinoa entraîne : (a) la stimulation de la prolifération des fibroblastes, (b) l'induction de l'expression du collagène I, collagène III et de l'élastine par les fibroblastes, (c) la modification des propriétés mécaniques des fibroblastes de vergetures rouge : effet relaxant transitoire et à long terme.

En stimulant la synthèse des différents composants de la matrice extracellulaire, le concentrât d'huile de Quinoa peut jouer un rôle important dans la restauration et la régultation de l'homéostasie dermique en cas d'altération (peau âgées, agressées, vergetures, cicatrices...).

### Exemple 5: extrait peptidique et osidique de quinoa (=peptides de quinoa) : activités biologiques

On a évalué l'activité des peptides de Quinoa, obtenus à l'exemple 3, sur les deux premiers mécanismes impliqués dans la réépithélisation cutanée (évaluation à deux concentrations : 0,05% MS et 0,1% MS) :
- la migration des Kératinocytes : (i) évaluation de l'effet sur l'expression des gènes composant la Laminine 5, (ii) évaluation de l'effet sur l'expression et la synthèse de la MMP-9 et (iii) évaluation de l'effet fonctionnel sur la migration des kératinocytes
- la prolifération des kératinocytes : (i) évaluation de l'effet sur la prolifération des kératinocytes, (ii) évaluation de l'effet sur la synthèse du KGF par les fibroblastes

### Matériel et Méthodes

### a. Etude de l'effet des peptides de Quinoa sur l'expression des gènes codant pour la Laminine 5 et la MMP-9 par RT-PCR

- Principe de la PCR Quantitative en temps réel : cf exemple 4
- Protocole :
   A J0, les kératinocytes ont été ensemencés en plaques 24 puits dans du milieu KGM-2. A J1, les cellules ont été traitées par le TGFβ1 à 5 ng/ml ou les peptides de Quinoa à 0,05 et 0,1% MS pendant 48 heures. A la fin du traitement des cellules, les ARN totaux ont été extraits (kit d'extraction RNeasy MiniKit ; Qiagen) puis dosés de façon quantitative en minichips à l'aide du système Experion (kit Experion RNA StdSens ; Biorad). Les ARN totaux ont ensuite été rétro-transcrits en cDNA (kit iScript cDNA Synthesis ; Biorad). Enfin, les cDNA néo-synthétisés relatifs au gène d'intérêt (gènes codant pour les 3 chaînes composant la laminine 5 : α3β3δ2 et MMP-9) ou aux gènes de référence (HPRT, GAPDH, YWHAZ, beta actin = normalisateurs) ont été amplifiés sélectivement par PCR en temps réel (iQ5, Biorad) en utilisant des amorces spécifiques des séquences cibles.
   L'expression des gènes de référence est analysée dans les mêmes échantillons que ceux pour lesquels l'expression des gènes d'intérêt est évaluée afin de normaliser les résultats et de s'assurer qu'ils sont bien le résultat de l'effet du traitement par les peptides de Quinoa.
- Analyse de résultats : les résultats sont normalisés par rapport au gène de référence le plus stable, cf exemple 4.

### b. Etude de la migration des Kératinocytes

Des supports de cultures plastiques sont revêtus par du collagène I ; un support contrôle est revêtu par de la gélatine (la migration sur gélatine est nettement plus faible que sur collagène natif). Les kératinocytes ont été ensemencés dans les boites revêtues et les cellules non adhérentes ont été éliminées après 6 heures d'incubation à 37°C et à 5% de CO₂. Le milieu de culture a été ensuite remplacé par du milieu contenant ou non les peptides de Quinoa à 0,1%. Après une nuit d'incubation, les divisions cellulaires ont été bloquées par incubation pendant 2 heures avec une solution de mitomycine C. Une cicatrice artificielle, reproductible a été réalisée sur les tapis cellulaires et, après lavages, le traitement a été renouvelé. Après 48 heures, les cellules ont été fixées et les noyaux marqués par le colorant fluorescent de Hoechst.

Des images numérisées ont été prises chaque jour. Une analyse de la migration des kératinocytes est effectuée entre l'image saisie au temps J0 (permettant la sélection de la cicatrice artificielle) et l'image « Hoechst » réalisée à J2 permettant le comptage du nombre de cellules migrantes.

### c. Etude de la prolifération des kératinocytes par la méthode au MTT

A J0, les Kératinocytes sont ensemencés en milieu KGM2 en plaque 96 puits. A J1, les cellules sont traitées par l'Acide Trans Rétinoique (ATRA) à 1 µM ou les peptides de Quinoa à 0,05% et 0,1% MS pendant 24 et 48 heures. En fin de traitement, la viabilité cellulaire est quantifiée par un test au MTT : après 3 heures de contact avec le MTT, les cristaux de formazan formés sont solubilisés par du DMSO et la densité optique, proportionnelle à la quantité de cellules métaboliquement actives donc vivantes, est lue à 570 nm contre le blanc (puits sans cellules).

### d. Dosage de la MMP-9 secrétée par les kératinocytes.

Les kératinocytes ont été ensemencés en plaque 24 puits ; après 24 heures d'incubation à 37°C, 5% de CO₂ les cellules ont été traitées par les peptides de Quinoa à 0,05% et 0,1% MS. Le TGFβ testé à 5ng/ml a été utilisé en tant témoin positif. Après 48 et 72 heures de traitement, la quantité de MMP-9 secreté par les cellules a été dosée dans le surnageant de culture à l'aide d'un kit ELISA (R&D Systems), selon le protocole préconisé par le fournisseur. En parallèle, la quantité de cellules vivantes par puits a été déterminée par un test colorimétrique au rouge neutre : la DO, proportionnelle à la quantité de cellules vivantes, est lue à 570 nm.

La quantité de MMP-9 est exprimée par cellules vivantes : (ng/ml)/ DO570 MTT.

### e. Dosage du KGF secrété par les Fibroblastes

Les Fibroblastes ont été ensemencés en plaque 24 puits dans du RPMI à 1% SVF ; après 24 heures d'incubation à 37°C, 5% de CO₂ les cellules ont été traitées par les peptides de Quinoa à 0,05% et 0,1% MS. L'IL1α (Sigma) à 100 ng/ml a été utilisé en tant témoin positif. Après 24 et 48 heures de traitement, la quantité du KGF relarguée par les fibroblastes a été dosée à l'aide d'un kit ELISA (R&D Systems), selon le protocole préconisé par le fournisseur. En parallèle, la quantité de cellules vivantes par puits a été déterminée par un test colorimétrique au rouge neutre : la Densité Optique (DO), proportionnelle à la quantité de cellules vivantes, est lue à 570 nm. En parallèle, la quantité de cellules vivantes par puits a été déterminée par un test colorimétrique au MTT : la DO, proportionnelle à la quantité de cellules vivantes, est lue à 570 nm.

La quantité du KGF est exprimée par cellules vivantes : pg/ml/ DO570 MTT. f. Statistiques : La significativité des résultats a été évaluée par un test t de Student.

### Résultats

### a. Expression de l'hétérotrimère α3β3δ2 de la laminine 5

Le contrôle génique du processus de ré-épithélisation implique plusieurs familles de facteurs de transcription ou de croissance, en particulier le TGF-β1 qui est libéré dès les premiers instants suivant une blessure. Dans les conditions physiologiques de la réépithélisation, il a été démontré que le TGF-β1 stimulait l'expression de la Laminine-5. Ce facteur de croissance est utilisé en tant que témoin positif pour étudier l'expression génique de cette protéine, afin de se rapprocher des conditions physiologiques.

L'analyse quantitative de la cinétique d'expression des ARNm des différentes chaînes composant la laminine 5 a été réalisée par PCR quantitative (Q-PCR) après 48 heures d'incubation avec le TGF-β1 à 5 ng/ml. Les résultats obtenus indiquent une induction très significative de l'expression des 3 gènes étudiés (p<0,01), et avec des facteurs d'induction atteignant 12 pour α3 et jusqu'à 17 pour γ2 (Tableau 10 : AB/C). L'ARNm β3 a lui aussi été induit à un niveau inférieur, de l'ordre de 5 fois.

On a étudié l'implication potentielle des peptides de Quinoa dans la régulation de l'expression des 3 gènes composant la Laminine 5. les résultats obtenus démontrent que les peptides de Quinoa stimulent de façon significative et dose dépendante l'expression de ces gènes, les facteurs d'induction atteignent 3 pour la chaîne α3, 2 pour la chaîne β3 et 2,6 pour la chaîne γ2.

**Tableau 10A : Expression génique de la chaîne α-3 de la laminine 5**

| | Moyenne dCt | Ecart Type | P | ddCt | QR | % d'induction |
|---|---|---|---|---|---|---|
| Contrôle | 1,435 | 0,659 | | 0,00 | 1,00 | |
| TGFβ à 5 ng/ml | -0,944 | 0,664 | p<0,01 | 3,69 | 12,93 | 1193 |
| Peptides de Quinoa 0,05% | 0,446 | 0,685 | p<0,01 | 1,49 | 2,82 | 182 |
| Peptides de Quinoa 0,1 % | 0,466 | 0,578 | p<0,01 | 1,64 | 3,12 | 212 |

**Tableau 10B : Expression génique de la chaîne β-3 de la laminine 5**

| | Moyenne dCt | Ecart Type | p | ddCt | QR | % d'induction |
|---|---|---|---|---|---|---|
| Contrôle | 1,435 | 0,659 | 0,00 | 1,00 | | |
| TGFβ à 5 ng/ml | -0,944 | 0,664 | p<0,01 | 2,38 | 5,20 | 420 |
| Peptides de Quinoa 0,05% | 0,446 | 0,685 | p<0,01 | 0,99 | 1,98 | 98 |
| Peptides de Quinoa 0, 1 % | 0,466 | 0,578 | p<0,01 | 0,97 | 1,96 | 96 |

**Tableau 10C : Expression génique de la chaîne γ-2 de la laminine 5**

| | Moyenne dCt | Ecart Type | p | ddCt | QR | % d'induction |
|---|---|---|---|---|---|---|
| Contrôle | 2,225 | 0,418 | | 0,00 | 1,00 | |
| TGFβ à 5 ng/ml | -1,925 | 0,453 | p<0,01 | 4,15 | 17,76 | 1676 |
| Peptides de Quinoa 0,05% | 1,502 | 0,489 | p<0,01 | 0,72 | 1,65 | 65 |
| Peptides de Quinoa 0,1 % | 0,839 | 0,336 | p<0,01 | 1,39 | 2,61 | 161 |

Tableau 10 : Expression génique des 3 chaînes composant la Laminine 5.

### b. Etude de l'expression de la MMP-9.

Parmi les protéases matricielles impliquées dans la ré-épithélisation, la MMP-9 joue un rôle particulièrement important. Elle est régulée positivement par le TGF-β1 et des cytokines pro-inflammatoires mais elle est exprimée également sur le site de la blessure par les kératinocytes migrant.

Comme montré dans le tableau 11, l'analyse des résultats de Q-PCR ont permis d'observer une induction précoce de l'expression du gène de la MMP-9 d'un facteur de 8 après 48 heures de stimulation par le TGFβ. En utilisant ce modèle, a également été mis en évidence l'effet des peptides de Quinoa sur la migration des kératinocytes au travers de leur action sur l'expression du gène MMP-9. Ainsi, les peptides de Quinoa stimulent significativement l'expression de la MMP-9 d'un facteur de 2,6 par rapport au contrôle sans traitement.

**Tableau 11 : Etude de l'expression génique de la MMP-9**

| | Moyenne dCt | Ecart Type | p | ddCt | QR | % d'induction |
|---|---|---|---|---|---|---|
| Contrôle | 6,08 | 0,84 | | 0,00 | 1,00 | |
| TGFβ à 5 ng/ml | 3,07 | 0,55 | p<0,01 | 3,01 | 8 | 807 |
| Peptides de Quinoa 0,05% | 5,06 | 0,57 | p<0,01 | 1,02 | 2 | 100 |
| Peptides de Quinoa 0,1 % | 4,68 | 0,74 | p<0,01 | 1,4 | 2,63 | 163 |

### c. Etude de la production de la protéine MMP-9

Après avoir étudié le profil d'expression génique de la MMP-9 en présence des peptides de Quinoa, on a vérifié que l'induction de l'expression du gène conduit également à une augmentation de la sécrétion de la protéine. L'influence des peptides de Quinoa sur la synthèse et la sécrétion de la MMP-9 par les kératinocytes, après un traitement de 48 et 72 heures, a donc été évaluée. Les résultats sont présentés dans le tableau 12. Ainsi, les peptides de Quinoa augmentent significativement la quantité de la MMP-9 secrétée par des kératinocytes (augmentation allant jusqu'à 55%).

**Tableau 12 : Dosage de la protéine MMP-9 dans les surnageants des kératinocytes.**

| | Traitement 48H | | | Traitement 72H | | |
|---|---|---|---|---|---|---|
| | Quantité MMP-9* | % A | Student t test | Quantité MMP-9* | % A | Student t test |
| Contrôle | 2,458 | | | 2,207 | | |
| TGFβ à 5 ng/ml | 11,604 | 372 | p<0,01 | 14,746 | 568 | p<0,01 |
| Peptides de Quinoa 0,05% | 2,799 | 14 | p<0,01 | 3,429 | 55 | p<0,01 |
| Peptides de Quinoa 0,1% | 2,988 | 22 | p<0,01 | 3,472 | 57 | p<0,01 |

| | | | | | | |
|---|---|---|---|---|---|---|
| * ng/ml/DO rouge neutre ; A = augmentation | | | | | | |

### d. Evaluation de l'effet fonctionnel des peptides de Quinoa sur la migration des kératinocytes

On a évalué l'effet fonctionnel des peptides de Quinoa sur la migration des kératinocytes dans une cicatrice artificielle après blocage de la prolifération cellulaire.

La comparaison visuelle des photos représentant les cellules traitées par les peptides de Quinoa avec les cellules non traités permet de conclure que les peptides de Quinoa favorisent la migration cellulaire : présence de plus de kératinocytes dans la cicatrice artificielle des cellules traitées par les peptides de Quinoa. Cette augmentation de la migration des kératinocytes a été quantifiée en comptant le nombre de cellules migrantes dans la plaie artificielle. Ainsi, les peptides de Quinoa augmentent de + 67% la migration des kératinocytes.

### e. Effet des peptides de Quinoa sur la prolifération des kératinocytes

Afin de vérifier si les peptides de Quinoa sont associés au processus de prolifération des kératinocytes (=seconde étape du mécanisme de réépithélialisation), un test de viabilité cellulaire au MTT a été réalisé, pour évaluer l'action directe des peptides de Quinoa sur la prolifération cellulaire. Comme montré dans le tableau 13, le traitement avec l'Acide Trans rétinoique (ATRA) = contrôle positif, induit un taux de prolifération de +36%. De la même façon, les peptides de Quinoa testés à 0,05 et 0,1% MS stimulent très significativement la prolifération des kératinocytes, avec une augmentation de l'ordre de + 20%.

**Tableau 13 : prolifération des kératinocytes**

| | Moyenne DO570 (MTT) | Ecart-Type | % de prolifération | Student t test |
|---|---|---|---|---|
| Contrôle Négatif | 1,003 | 0,050 | 100 | |
| ATRA à 1µM | 1,361 | 0,140 | 136 | p<0,01 |
| Peptides de Quinoa 0,05% | 1,156 | 0,075 | 115 | P<0,01 |
| Peptides de Quinoa 0, 1 % | 1,201 | 0,071 | 120 | p<0,01 |

### f. Etude de la synthèse du KGF par les fibroblastes

Au cours de la ré-épithélisation, le processus de prolifération des kératinocytes s'effectue sous l'influence de nombreux facteurs qui peuvent être sécrétés par les kératinocytes ou par les fibroblastes du derme : production du KGF (Kératinocyte growth Factor). En réponse au KGF surexprimé par les fibroblastes, les kératinocytes se multiplient et recouvrent ainsi plus rapidement la lésion. L'effet des peptides de Quinoa sur la sécrétion de ce facteur par les fibroblastes dermiques, après un traitement de 24 et 48 heures, a été évalué. Dans le tableau 14, on confirme que la synthèse du KGF augmente en présence de l'IL1α. D'autre part, les peptides de Quinoa testés à 0,05 et 0,1% MS stimulent de manière très significative la sécrétion du KGF par les fibroblastes. Cette stimulation est dose dépendante et très importante dès 24 heures de traitement (multiplication par 5 et 7 par rapport au contrôle sans traitement).

**Tableau 14 : Dosage du KGF dans les surnageants des fibroblastes.**

| | Traitement 24H | | | Traitement 48H | | |
|---|---|---|---|---|---|---|
| | Quantité de KGF* | %A | Student t test | Quantité de KGF* | %A | Student t test |
| Contrôle | 27,853 | | | 80,884 | | |
| TGFβ à 5 ng/ml | 147,886 | 431 | p<0,01 | 352,387 | 336 | p<0,01 |
| Peptides de Quinoa 0,05% | 135,454 | 386 | p<0,01 | 148,744 | 84 | p<0,01 |
| Peptides de Quinoa 0, 1 % | 194,650 | 599 | p<0,01 | 160,405 | 98 | p<0,01 |

| | | | | | | |
|---|---|---|---|---|---|---|
| * pg/ml/DO rouge neutre; A = augmentation | | | | | | |

**Conclusion :** L'ensemble des études présentées ici ont permit de mettre en évidence le rôle des peptides de Quinoa dans la cicatrisation épidermique. En stimulant les deux processus, les peptides de Quinoa permettent une restauration efficace et rapide de l'intégrité de la peau.

## Revendications

1. Composition cosmétique, dermatologique ou nutraceutique comprenant un extrait lipidique de graines de quinoa et le cas échéant un excipient approprié, **caractérisée en ce que** ledit extrait lipidique de quinoa est un insaponifiable.

2. Composition selon la revendication 1, **caractérisée en ce que** l'extrait lipidique est un insaponifiable, ayant les spécifications suivantes :
| | |
|---|---|
| Teneur en Tocophérols (g/100g) | 1,5 - 3,5 |
| Teneur en Stérols (g/100g) | 20,0 - 50,0 |
| Teneur en Squalène (g/100 g) | 20,0 - 50,0 |

3. Procédé de préparation d'un insaponifiable de quinoa, comprenant
- une étape de préparation d'une huile de quinoa concentrée en sa fraction insaponifiable comprenant une étape de distillation moléculaire d'une huile raffinée de quinoa, puis
- une étape de saponification de ladite huile de quinoa concentrée en sa fraction insaponifiable, puis
- une extraction de cet insaponifiable à l'aide d'un solvant approprié.

4. Procédé selon la revendication 3, **caractérisé en ce que** l'étape de distillation moléculaire est réalisée en utilisant un dispositif choisi parmi les distillateurs moléculaires de type centrifuge et les dispositifs moléculaires de type à film raclé

5. Composition selon l'une quelconque des revendications 1 à 3 ou huile raffinée ayant les spécifications suivantes :
| Coupe grasse (% en poids par rapport au poids total de l'huile) | |
|---|---|
| C14 | ≤ 1,0 |
| C16 | 5,0-12,0 |
| C16' | ≤ 1,0 |
| C18 | ≤ 2,0 |
| C18' | 20,0-35,0 |
| C18" | 40,0-60,0 |
| C18'" | 2,0-13,0 |
| C20 | ≤ 1,0 |
| C20' | ≤ 3,0 |
| C22 | ≤ 1,0 |
| C22' | ≤ 3,0 |
| C24 | ≤ 1,0 |
| Teneur en Tocophérols (mg/100g) | 4 - 200 |
| Teneur en Stérols (g/100g) | 0,5 - 3,0 |
| Teneur en Squalène (g/100 g) | 0,5 - 3,0 |
| Teneur en Insaponifiable (g/100 g) | 2,0-6,0 |
Ou huile concentrée en sa fraction insaponifiable, ayant les spécifications suivantes :
| Coupe grasse (% en poids par rapport au poids total de l'huile) | |
|---|---|
| C14 | ≤ 1,0 |
| C16 | 5,0-12,0 |
| C16' | ≤ 1,0 |
| C18 | ≤ 2,0 |
| C18' | 20,0-35,0 |
| C18" | 40,0-60,0 |
| C18'" | 2,0-13,0 |
| C20 | ≤ 1,0 |
| C20' | ≤ 3,0 |
| C22 | ≤ 1,0 |
| C22' | ≤ 3,0 |
| C24 | ≤1.0 |
| Teneur en Tocophérols (mg/100g) | 40-5000 |
| Teneur en Stérols (g/100g) | 3,0-20,0 |
| Teneur en Squalène (g/100 g) | 2,0 - 40,0 |
| Teneur en Insaponifiable (g/100 g) | 5,0 - 50,0 |
pour son utilisation dans la prévention et le traitement des réactions ou pathologies allergiques, inflammatoires, irritatives de la peau et/ou des muqueuses et/ou des phanères immatures, normales ou matures.

6. Composition comprenant une huile concentrée en sa fraction insaponifiable, ayant les spécifications suivantes :
| Coupe grasse (% en poids par rapport au poids total de l'huile) | |
|---|---|
| C14 | ≤ 1,0 |
| C16 | 5,0 -12,0 |
| C16' | ≤1,0 |
| C18 | ≤ 2,0 |
| C18' | 20,0-35,0 |
| C18" | 40,0 - 60,0 |
| C18"' | 2,0 - 13.0 |
| C20 | ≤ 1,0 |
| C20' | ≤ 3,0 |
| C22 | ≤ 1,0 |
| C22' | ≤ 3,0 |
| C24 | ≤ 1,0 |
| Teneur en Tocophérols (mg/100g) | 40-5000 |
| Teneur en Stérols (g/100g) | 3.0-20,0 |
| Teneur en Squalène (g/100 g) | 2,0-40,0 |
| Teneur en Insaponifiable (g/100 g) | 5,0-50,0 |
et le cas échéant un excipient approprié pour son utilisation :
- dans la prévention et/ou le traitement des plaies profondes,
- pour favoriser la cicatrisation, ou
- dans la prévention et/ou le traitement des atrophies sous-cutanées du derme.

7. Méthode de traitement cosmétique visant à prévenir ou à traiter le vieillissement cutané ou les vergetures, **caractérisée en ce qu'**on utilise une composition comprenant une huile concentrée en sa fraction insaponifiable, avant les spécifications suivantes :
| Coupe grasse (% en poids par rapport au poids total de l'huile) | |
|---|---|
| C14 | ≤ 1,0 |
| C16 | 5,0-12,0 |
| C16' | ≤ 1,0 |
| C18 | ≤ 2,0 |
| C18' | 20,0-35,0 |
| C18" | 40,0-60,0 |
| C18"' | 2,0-13,0 |
| C20 | ≤ 1,0 |
| C20' | ≤ 3,0 |
| C22 | ≤ 1,0 |
| C22' | ≤ 3,0 |
| C24 | ≤ 1,0 |
| Teneur en Tocophérols (mg/100g) | 40-5000 |
| Teneur en Stérols (g/100g) | 3,0-20,0 |
| Teneur en Squalène (g/100 g) | 2,0-40,0 |
| Teneur en Insaponifiable (g/100 g) | 5,0-50,0 |
et le cas échéant un excipient approprié

8. Composition comprenant un extrait peptidique et osidique de graines de quinoa, susceptible d'être obtenu par un procédé comprenant les étapes successives suivantes :
a) à partir de graines de quinoa, extraction d'une huile brute et d'un tourteau et récupération dudit tourteau ;
b) lavage dudit tourteau par l'eau ou un mélange hydroalcoolique pour ne conserver que la partie protéique, puis
c) solubilisation des protéines ;
d) concentration des protéines puis hydrolyse desdites protéines en peptides ;
e) purification et récupération de l'extrait peptidique et osidique,
et le cas échéant un excipient approprié,
pour son utilisation :
- pour favoriser la cicatrisation ;
- dans la prévention et/ou le traitement de l'acné ;
- pour réguler les troubles de la pigmentation ; ou
- dans la prévention et/ou le traitement des hirsutismes ou des dermites séborrhéiques.

9. Composition selon la revendication 8, **caractérisée en ce que** l'extrait peptidique et osidique est constitué de 25 à 90% en poids de peptides et de 10 à 50% en poids de sucres, les pourcentages étant exprimés en poids par rapport au poids total dudit extrait peptidique et osidique.

10. Méthode de traitement cosmétique des phanères, en particulier des cheveux, des poils, ou des ongles, de l'alopécie, des pellicules, ou des peaux normales, sèches, grasses, mixtes, déshydratées, âgées ou sensibles, **caractérisée en ce qu'**elle consiste à administrer une composition comprenant un extrait peptidique et osidique de graines de quinoa, susceptible d'être obtenu par un procédé comprenant les étapes successives suivantes :
a) à partir de graines de quinoa, extraction d'une huile brute et d'un tourteau et récupération dudit tourteau ;
b) lavage dudit tourteau par l'eau ou un mélange hydroalcoolique pour ne conserver que la partie protéique, puis
c) solubilisation des protéines ;
d) concentration des protéines puis hydrolyse desdites protéines en peptides ;
e) purification et récupération de l'extrait peptidique et osidique.

11. Méthode de traitement cosmétique selon la revendication 10, **caractérisée en ce que** l'extrait peptidique et osidique est constitué de 25 à 90% en poids de peptides et de 10 à 50% en poids de sucres, les pourcentages étant exprimés en poids par rapport au poids total dudit extrait peptidique et osidique.

## Claims

1. Cosmetic, dermatological or nutraceution composition comprising a lipid extract of quinoa grains and, if necessary, a suitable excipient, **characterised in that** said lipid quinoa extract is an unsaponifiable.

2. Composition according to claim 1, **characterised in that** the lipid extract is an unsaponifiable, having the following specifications:
| | |
|---|---|
| Tocopherol content (g/100g) | 1.5-3.5 |
| Sterol content (g/100g) | 20.0-50.0 |
| Squalene content (g/100g) | 20.0-50.0 |

3. Method for preparing a quinoa unsaponifiable, comprising
- a step for preparing quinoa oil concentrated in the unsaponifiable fraction thereof, comprising a step for molecular distillation of a refined quinoa oil, and then
- a step for saponifying said quinoa oil concentrated in the unsaponifiable fraction thereof, and then
- extracting this unsaponifiable with a suitable solvent.

4. Method according to claim 3, **characterised in that** the molecular distillation step is carried out with the use of a device selected from molecular distillers of the centrifuge type and molecular devices of the type with a scraped film.

5. Composition according to any or claims 1 to 3, or a refined oil having the following specifications:
| Fat cut (% by weight based on the total weight of the oil) | |
|---|---|
| C14 | ≤ 1.0 |
| C16 | 5.0-12.0 |
| C16' | ≤ 1.0 |
| C18 | ≤ 2.0 |
| C18' | 20.0-35.0 |
| C18" | 40.0-60.0 |
| C18"' | 2.0-13.0 |
| C20 | ≤ 1.0 |
| C20' | ≤ 3.0 |
| C22 | ≤ 1.0 |
| C22' | ≤ 3.0 |
| C24 | ≤ 1.0 |
| Tocopherol content (mg/100g) | 4-200 |
| Sterol content (g/100g) | 0.5-3.0 |
| Squalene content (g/100g) | 0.5-3.0 |
| Unsaponifiable content (g/100g) | 2.0-6.0 |
Or oil concentrated in the unsaponifiable fraction thereof, having the following specifications:
| Fat cut (% by weight based on the total weight of the oil) | |
|---|---|
| C14 | ≤ 1.0 |
| C16 | 5.0-12.0 |
| C16' | ≤ 1.0 |
| C18 | ≤ 2.0 |
| C18' | 20.0-35.0 |
| C18" | 40.0-60.0 |
| C18'" | 2.0-13.0 |
| C20 | ≤ 1.0 |
| C20' | ≤ 3.0 |
| C22 | ≤ 1.0 |
| C22' | ≤ 3.0 |
| C24 | ≤ 1.0 |
| Tocopherol content (mg/100g) | 40-5000 |
| Sterol content (g/100g) | 3.0-20.0 |
| Squalene content (g/100g) | 2.0-40.0 |
| Unsaponifiable content (g/100g) | 5.0-50.0 |
for use in the prevention and treatment of allergic, inflammatory, irritative pathologies or reactions of the skin and/or mucosas and/or immature, normal or mature integuments.

6. Composition comprising an oil concentrated in the unsaponifiable fraction thereof, having the following specifications:
| Fat cut (% by weight based on the total weight of the oil) | |
|---|---|
| C14 | ≤ 1.0 |
| C16 | 5.0-12.0 |
| C16' | ≤ 1.0 |
| C18 | ≤ 2.0 |
| C18' | 20.0-35.0 |
| C18" | 40.0-60.0 |
| C18"' | 2.0-13.0 |
| C20 | ≤ 1.0 |
| C20' | ≤ 3.0 |
| C22 | ≤ 1.0 |
| C22' | ≤ 3.0 |
| C24 | ≤ 1.0 |
| Tocopherol content (mg/100g) | 40-5000 |
| Sterol content (g/100g) | 3.0-20.0 |
| Squalene content (g/100g) | 2.0-40.0 |
| Unsaponifiable content (g/100) | 5.0-50.0 |
and, if necessary, a suitable excipient, for use thereof for:
- preventing and/or treating deep wounds,
- promoting healing, or
- preventing and/or treating subcutaneous atrophies of the dermis.

7. Cosmetic treatment method for preventing or treating skin ageing or stretch marks, **characterised in that** a composition comprising an oil concentrated in the unsaponifiable fraction thereof, having the following specifications:
| Fat cut (% by weight based on the total weight of the oil) | |
|---|---|
| C14 | ≤ 1.0 |
| C16 | 5.0-12.0 |
| C16' | ≤ 1.0 |
| C18 | ≤ 2.0 |
| C18' | 20.0-35.0 |
| C18" | 40.0-60.0 |
| C18'" | 2.0-13.0 |
| C20 | ≤ 1.0 |
| C20' | ≤ 3.0 |
| C22 | ≤ 1.0 |
| C22' | ≤ 3.0 |
| C24 | ≤ 1.0 |
| Tocopherol content (mg/100g) | 40-5000 |
| Sterol content (g/100g) | 3.0-20.0 |
| Squalene content (g/100g) | 2.0-40.0 |
| Unsaponifiable content (g/100g) | 5.0-50.0 |
and, if necessary, a suitable excipient, are used.

8. Composition comprising a peptide and oside extract of quinoa grains, obtained by means of a method comprising the following successive steps:
a) starting with quinoa grains, extraction of a raw oil and of a cake and recovery of said cake;
b) washing said cake with water or with a water/alcohol mixture in order to only retain the protein portion, and then
c) solubilising the proteins;
d) concentrating the proteins and then hydrolysing said proteins into peptides;
e) purifying and recovering the peptide and oside extract,
and, if necessary, a suitable excipient,
for the use thereof for:
- promoting healing;
- preventing and/or treating acne;
- regulating pigmentation disorders; or
- preventing and/or treating hirsutism or seborrhoeic dermatitis.

9. Composition according to claim 8, **characterised in that** the peptide and oside extract consists of 25 to 90% by weight of peptides and from 10 to 50% by weight of sugars, the percentages being expressed relatively to the total weight of said peptide and oside extract.

10. Cosmetic treatment method for integuments, particularly hair on the head or body, or nails, alopecia, dandruff, or normal, dry, greasy, mixed, dehydrated, aged or sensitive skin, **characterised in that** it consists of administering a composition comprising a peptide and oside extract of quinoa grains, suitable for being obtained by means of a method comprising the following successive steps:
a) starting with quinoa grains, extraction of a raw oil and of a cake and recovery of said cake;
b) washing said cake with water or with a water/alcohol mixture in order to only retain the protein portion, and then
c) solubilising the proteins;
d) concentrating the proteins and then hydrolysing said proteins into peptides;
e) purifying and recovering the peptide and oside extract.

11. Cosmetic treatment method according to claim 10, **characterised in that** the peptide and oside extract consists of 25 to 90% by weight of peptides and from 10 to 50% by weight of sugars, the percentages being expressed relatively to the total weight of said peptide and oside extract.

## Patentansprüche

1. Kosmetische, dermatologische oder nutrazeutische Zusammensetzung, umfassend einen Lipidextrakt aus Quinoa-Körnern und gegebenenfalls einen geeigneten Exzipienten, **dadurch gekennzeichnet, dass** der Quinoa-Lipidextrakt eine nicht verseifbare Substanz ist.

2. Zusammensetzung nach Anspruch 1, **dadurch gekennzeichnet, dass** der Lipidextrakt eine nicht verseifbare Substanz ist, welche die folgenden Merkmale aufweist:
| | |
|---|---|
| Gehalt an Tocopherolen (g/100 g) | 1,5 - 3,5 |
| Gehalt an Sterolen (g/100 g) | 20,0 - 50,0 |
| Gehalt an Squalen (g/100 g) | 20,0 - 50,0 |

3. Verfahren zur Herstellung einer nicht verseifbaren Substanz aus Quinoa, umfassend
- einen Schritt des Herstellens eines in seiner nicht verseifbaren Fraktion konzentrierten Quinoa-Öls, umfassend einen Schritt der Molekulardestillation eines raffinierten Quinoa-Öls, gefolgt von
- einem Schritt des Verseifens des in seiner nicht verseifbaren Fraktion konzentrierten Quinoa-Öls, gefolgt von
- einer Extraktion der nicht verseifbaren Substanz mit Hilfe eines geeigneten Lösungsmittels.

4. Verfahren nach Anspruch 3, **dadurch gekennzeichnet, dass** der Schritt der Molekulardestillation unter Verwendung einer Vorrichtung, ausgewählt aus Molekulardestillationsapparaten vom Zentrifugentyp und molekularen Vorrichtungen vom Rakelschichttyp, durchgeführt wird.

5. Zusammensetzung nach einem der Ansprüche 1 bis 3 oder raffiniertes Öl, welches die folgenden Merkmale aufweist:
| Fettanteil (Gew.-% bezogen auf das Gesamtgewicht des Öls) | |
|---|---|
| C14 | ≤ 1,0 |
| C16 | 5,0-12,0 |
| C16' | ≤ 1,0 |
| C18 | ≤ 2,0 |
| C18' | 20,0-35,0 |
| C18" | 40,0-60,0 |
| C18'" | 2,0-13,0 |
| C20 | ≤ 1,0 |
| C20' | ≤ 3,0 |
| C22 | ≤ 1,0 |
| C22' | ≤ 3,0 |
| C24 | ≤ 1,0 |
| Gehalt an Tocopherolen (mg/100 g) | 4-200 |
| Gehalt an Sterolen (g/ 100 g) | 0,5-3,0 |
| Gehalt an Squalen (g/100 g) | 0,5-3,0 |
| Gehalt an nicht verseifbarer Substanz (g/100 g) | 2,0-6,0 |
oder in seiner nicht verseifbaren Fraktion konzentriertes Öl, welches die folgenden Merkmale aufweist:
| Fettanteil (Gew.-% bezogen auf das Gesamtgewicht des Öls) | |
|---|---|
| C14 | ≤ 1,0 |
| C16 | 5,0-12,0 |
| C16' | ≤ 1,0 |
| C18 | ≤ 2,0 |
| C18' | 20,0-35,0 |
| C18" | 40,0-60,0 |
| C18"' | 2,0-13,0 |
| C20 | ≤ 1,0 |
| C20' | ≤ 3,0 |
| C22 | ≤ 1,0 |
| C22' | ≤ 3,0 |
| C24 | ≤ 1,0 |
| Gehalt an Tocopherolen (mg/100 g) | 40-5000 |
| Gehalt an Sterolen (g/100 g) | 3,0-20,0 |
| Gehalt an Squalen (g/100 g) | 2,0-40,0 |
| Gehalt an nicht verseifbarer Substanz (g/100 g) | 5,0-50,0 |
zur Verwendung bei der Prävention und Behandlung von allergischen, entzündlichen, irritativen Reaktionen oder Erkrankungen der Haut und/oder der Schleimhäute und/oder unreifer, normaler oder reifer Phanere.

6. Zusammensetzung, umfassend ein in seiner nicht verseifbaren Fraktion konzentriertes Öl, welches die folgenden Merkmale aufweist:
| Fettanteil (Gew.-% bezogen auf das Gesamtgewicht des Öls) | |
|---|---|
| C14 | ≤ 1,0 |
| C16 | 5,0-12,0 |
| C16' | ≤ 1,0 |
| C18 | ≤ 2,0 |
| C18' | 20,0-35,0 |
| C18" | 40,0-60,0 |
| C18'" | 2,0-13,0 |
| C20 | ≤ 1,0 |
| C20' | ≤ 3,0 |
| C22 | ≤ 1,0 |
| C22' | ≤ 3,0 |
| C24 | ≤ 1,0 |
| Gehalt an Tocopherolen (mg/ 100 g) | 40 - 5000 |
| Gehalt an Sterolen (g/100 g) | 3,0 - 20,0 |
| Gehalt an Squalen (g/100 g) | 2,0 - 40,0 |
| Gehalt an nicht verseifbarer Substanz (g/100 g) | 5,0 - 50,0 |
und gegebenenfalls einen geeigneten Exzipienten zur Verwendung:
- bei der Prävention und/oder Behandlung von tiefen Wunden,
- zur Begünstigung der Narbenbildung oder
- bei der Prävention und/oder Behandlung von subkutanen Atrophien der Lederhaut.

7. Verfahren zur kosmetischen Behandlung zur Prävention oder Behandlung der Hautalterung oder von Schwangerschaftsstreifen, **dadurch gekennzeichnet, dass** man eine Zusammensetzung, umfassend ein in seiner nicht verseifbaren Fraktion konzentriertes Öl, welches die folgenden Merkmale aufweist:
| Fettanteil (Gew.-% bezogen auf das Gesamtgewicht des Öls) | |
|---|---|
| C14 | ≤ 1,0 |
| C16 | 5,0-12,0 |
| C16' | ≤ 1,0 |
| C18 | ≤ 2,0 |
| C18' | 20,0-35,0 |
| C18" | 40,0-60,0 |
| C18'" | 2,0-13,0 |
| C20 | ≤ 1,0 |
| C20' | ≤ 3,0 |
| C22 | ≤ 1,0 |
| C22' | ≤ 3,0 |
| C24 | ≤ 1,0 |
| Gehalt an Tocopherolen (mg/100 g) | 40-5000 |
| Gehalt an Sterolen (g/100 g) | 3,0-20,0 |
| Gehalt an Squalen (g/100 g) | 2,0-40,0 |
| Gehalt an nicht verseifbarer Substanz (g/100 g) | 5,0 - 50,0 |
und gegebenenfalls einen geeigneten Exzipienten verwendet.

8. Zusammensetzung, umfassend einen Peptid- und Osidextrakt von Quinoa-Körnern, erhältlich durch ein Verfahren, das die folgenden aufeinanderfolgenden Schritte umfasst:
a) ausgehend von Quinoa-Körnern, Extraktion eines Rohöls und eines Ölkuchens und Gewinnen des Ölkuchens;
b) Waschen des Ölkuchens mit Wasser oder einem hydroalkoholischen Gemisch, um nur den Proteinanteil zu erhalten, gefolgt von
c) Löslichmachen der Proteine;
d) Konzentrieren der Proteine, gefolgt von Hydrolyse der Proteine zu Peptiden;
e) Reinigen und Gewinnen des Peptid- und Osidextrakts;
und gegebenenfalls einen geeigneten Exzipienten
zur Verwendung:
- zur Begünstigung der Narbenbildung;
- bei der Prävention und/oder Behandlung von Akne;
- zur Regulierung von Pigmentstörungen; oder
- bei der Prävention und/oder Behandlung von Hirsutismen oder seborrhoischer Dermatitis.

9. Zusammensetzung nach Anspruch 8, **dadurch gekennzeichnet, dass** der Peptid- und Osidextrakt aus 25 bis 90 Gew.-% Peptiden und 10 bis 50 Gew.-% Zuckern besteht, wobei die Prozentsätze in Gewicht bezogen auf das Gesamtgewicht des Peptid- und Osidextrakts ausgedrückt sind.

10. Verfahren zur kosmetischen Behandlung von Phaneren, insbesondere Kopfhaaren, Körperhaaren oder Nägeln, von Alopezie, Häutchen oder normaler, trockener, fettiger, Misch-, dehydratisierter, gealterter oder empfindlicher Haut, **dadurch gekennzeichnet, dass** es darin besteht, eine Zusammensetzung, umfassend einen Peptid- und Osidextrakt von Quinoa-Körnern, erhältlich durch ein Verfahren, das die folgenden aufeinanderfolgenden Schritte umfasst:
a) ausgehend von Quinoa-Körnern, Extraktion eines Rohöls und eines Ölkuchens und Gewinnen des Ölkuchens;
b) Waschen des Ölkuchens mit Wasser oder einem hydroalkoholischen Gemisch, um nur den Proteinanteil zu erhalten, gefolgt von
c) Löslichmachen der Proteine;
d) Konzentrieren der Proteine, gefolgt von Hydrolyse der Proteine zu Peptiden;
e) Reinigen und Gewinnen des Peptid- und Osidextrakts
zu verabreichen.

11. Verfahren zur kosmetischen Behandlung nach Anspruch 10, **dadurch gekennzeichnet, dass** der Peptid- und Osidextrakt aus 25 bis 90 Gew.-% Peptiden und 10 bis 50 Gew.-% Zuckern besteht, wobei die Prozentsätze in Gewicht bezogen auf das Gesamtgewicht des Peptid- und Osidextrakts ausgedrückt sind.
